(19) Europäisches Patentamt
European Patent Office
Office européen des brevets

(11) Publication number: **0 101 076**
**B1**

(12) # EUROPEAN PATENT SPECIFICATION

(45) Date of publication of patent specification: **04.01.89**

(51) Int. Cl.$^4$: **C 12 P 41/00,** C 07 D 263/24, C 12 P 17/14

(21) Application number: **83107962.9**

(22) Date of filing: **11.08.83**

(54) **Process for production of optically active oxazolidinone derivative.**

(30) Priority: **13.08.82 JP 141575/82**
**28.10.82 JP 190585/82**

(43) Date of publication of application:
**22.02.84 Bulletin 84/08**

(45) Publication of the grant of the patent:
**04.01.89 Bulletin 89/01**

(84) Designated Contracting States:
**BE CH DE FR GB IT LI NL SE**

(56) References cited:

**AGRICULTURAL AND BIOLOGICAL CHEMISTRY, vol. 39, no. 1, January 1975, pages 89-96; TAKAYUKI ORITANI et al.: "Microbial resolution of some racemic monocyclic alcohols"**

(73) Proprietor: **KANEGAFUCHI KAGAKU KOGYO KABUSHIKI KAISHA**
**2-4 Nakanoshima 3-chome**
**Kita-ku Osaka-shi Osaka-fu (JP)**

(72) Inventor: **Hamaguchi, Shigeki**
**Kodan 42-501 Minatojima Nakamachi**
**Chuo-ku Kobe-shi Hyogo-ken (JP)**
Inventor: **Asada, Masanori**
**3-5-21 Okihama-cho Takasago-cho**
**Takasago-shi Hyogo-ken (JP)**
Inventor: **Yamamura, Hiroshi**
**2-63 Okihama-cho Takasago-cho**
**Takasago-shi Hyogo-ken (JP)**
Inventor: **Hasegawa, Junzo**
**13-4 Takaoka 2-chome Okubo-cho**
**Akashi-shi Hyogo-ken (JP)**
Inventor: **Kawaharada, Hajime**
**2183-4 Shinzaike Hiraoka-cho**
**Kakogawa-shi Hyogo-ken (JP)**
Inventor: **Watanabe, Kiyoshi**
**15-41 Matsugaoka 5-chome**
**Akashi-shi Hyogo-ken (JP)**

(74) Representative: **Türk, Gille, Hrabal**
**Bruckner Strasse 20**
**D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 101 076 B1**

Courier Press, Leamington Spa, England.

## Description

The present invention relates to a process for production of optically active oxazolidinone derivatives. More particularly, the present invention relates to production of an (S)-(+)-3-alkyl-5-acyloxymethyloxazolidin-2-one having the formula [(S)—II]:

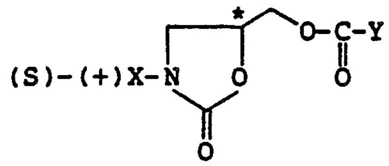

[(S)-II]

wherein X is a lower alkyl group having 1 to 6 carbon atoms and Y is a substituted or unsubstituted alkyl group, an alkenyl group, or a substituted or unsubstituted aromatic hydrocarbon group, and an (S)-(+)-3-alkyl-5-hydroxymethyloxazolidin-2-one having the formula [(S)—I]:

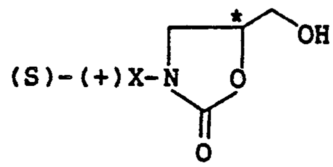

[(S)-I]

wherein X is as described above, by utilizing the action of a microorganism or an enzyme derived from the microorganism or an animal tissue, i.e. the microorganism and enzyme having a stereoselective esterase activity.

The compounds [(S)—II] and [(S)—I] are important intermediates for preparing optically active β-adrenergic blocking agents (hereinafter referred to as "β-blockers").

As processes for preparing optically active β-blockers, there hitherto has been known the following methods:

(1) A method in which a racemate of β-blocker is optically resolved, as disclosed in Japanese Unexamined Patent Publication No. 149233/1980 and U.S. Patent No. 3,655,663.

(2) A method in which an optically active β-blocker is prepared from an optically active 3-alkylamino-1,2-propanediol obtained by optical resolution of the racemate thereof, as disclosed in Japanese Unexamined Patent Publication No. 118,711/1976.

(3) A method in which an optically active β-blocker is prepared from D-mannitol via D-glycerol acetonide, as disclosed in Journal of Organic Chemistry, *41* (19), 3121 to 3124 (1976); Chemical and Pharmaceutical Bulletin, *29* (12), 3593 to 3600 (1981).

However, the method (1) is disadvantageous from the economical point of view because the final product of the racemate is optically resolved. The method (2) cannot easily provide an optically active 3-alkylamino-1,2-propanediol in high yield and high purity by using an optical resolution, because the 3-alkylamino-1,2-propanediol is hygroscopic and poor in crystallization. And the method (3) requires a large amount of lead tetraacetate for preparing D-glycerol acetonide from D-mannitol, and accordingly is not suitable for the industrial production. Thus, these processes include economical or practical problems, and there has not been industrially advantageous process for the production of optically active β-blockers.

On the other hand, the following reaction pathway is known as a rational synthetic pathway for preparing optically active β-blockers, as disclosed in Yoshisuke Tsuda et al: Chemical and Pharmaceutical Bulletin, *29* (12), 3593 to 3600 (1981); Canadian Patent No. 965,787.

D-mannitol→→D-glyceraldehyde acetonide→→

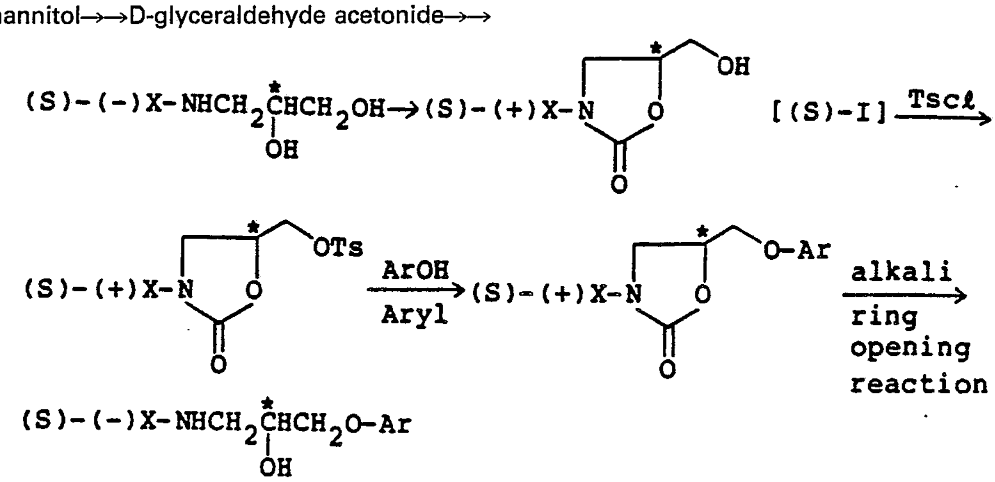


[optically active β-blocker]

wherein X is as described above, Ar is an aryl derivative.

It is an object of the present invention to provide novel and simple processes for producing the compound [(S)—I]. Another object of the present invention is to provide novel and simple processes for producing the compound [(S)—II] which is a useful intermediate for producing the compound. [(S)—I], and to provide novel starting products (R,S)—II.

The present inventors have considered that a (R,S)-(±)-3-alkyl-5-acyloxymethyloxazolidin-2-one can be easily prepared from a (R,S)-(±)-3-alkyl-5-hydroxymethyloxazolidin-2-one by esterifying and that an optically active (S)-(+)-3-alkyl-5-hydroxymethyloxazolidin-2-one having the formula [(S)—I] can be prepared from the above (R,S)-(±)-3-alkyl-5-acyloxymethyloxazolidin-2-one by the stereoselective hydrolysis using a microorganism or an enzyme. EP—A—0 123 719 describes and claims further processes for preparing the starting products (R,S)-II. As a result of various studies, the present inventors have now found microorganisms and enzymes having a stereoselective esterase activity capable of asymmetrically hydrolyzing the (R,S)-(±)-3-alkyl-5-acyloxymethyloxazolidin-2-one so as to substantially produce an optically active (R)-(−)-3-alkyl-5-hydroxymethyloxazolidin-2-one, and further found that the compound [(S)—I] can be obtained by isolating the unreacted compound [(S)—II] through a simple procedure and hydrolyzing the compound [(S)—II].

In accordance with the present invention, there can be provided a process for producing an (S)-(+)-3-alkyl-5-acyloxymethyloxazolidin-2-one which comprises subjecting an (R,S)-(±)-3-alkyl-5-acyloxymethyl-oxazolidin-2-one having the formula [(R,S)—II]:

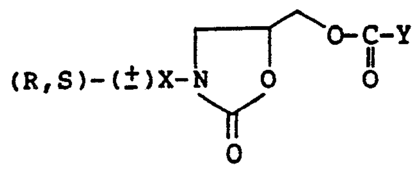

[(R,S)-II]

wherein X is a lower alkyl group having 1 to 6 carbon atoms and Y is a substituted or unsubstituted alkyl group, and alkenyl group, or a substituted or unsubstituted aromatic hydrocarbon group, to the action of a microorganism or an enzyme derived from the microorganism or an animal tissue; i.e. the microorganism and enzyme having a stereoselective esterase activity capable of asymmetrically hydrolyzing the compound [(R,S)—II] so as to produce substantially an (R)-(−)-3-alkyl-5-hydroxymethyloxazolidin-2-one having the formula [(R)—I]:

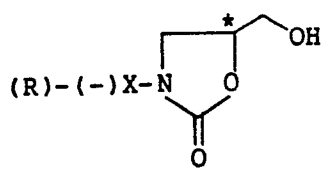

[(R)-I]

wherein X is as defined above, and isolating the unreacted (S)-(+)-3-alkyl-5-acyloxymethyloxazolidin-2-one having the formula [(S)—II].

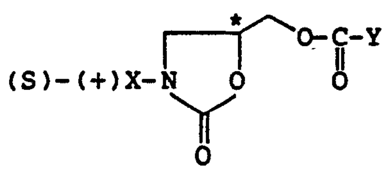

[(S)-II]

The optically active compound [(S)—II] is recovered by various methods such as extraction with an organic solvent, column chromatography and fractional distillation. The compound [(S)—II] can be easily converted into the optically active compound [(S)—I] by hydrolysis. Accordingly, the present invention also provides a process for preparing the optically active compound [(S)—I].

The summary of the pathway for obtaining the compound [(S)—I] is as follows:

[(R,S)-II] —enzymatic and asymmetric hydrolysis→ [(S)-II] —hydrolysis→

[(S)-I]

According to the present invention, optically active β-blockers can be synthesized in high purity and inexpensive by conventional methods by using the compound [(S)—I].

The substituent group defined as X in the formula [(R,S)—I] or [(R,S)—II] is a lower alkyl group having 1 to 6 carbon atoms, such as methyl, ethyl, propyl, isopropyl, butyl or t-butyl group, preferably, isopropyl or t-butyl group. The substituent group defined as Y in the formula [(R,S)—II] is a substituted or unsubstituted

alkyl group having 1 to 17, an alkenyl group having 2 to 8 carbon atoms, or a substituted or unsubstituted aromatic hydrocarbon group. More particularly, examples of the above Y are, for instance, an unsubstituted alkyl group such as methyl, ethyl, propyl or butyl group; a substituted alkyl group having a substituent group such as halogen, hydroxyl or alkoxy group, e.g. chloromethyl, dichloromethyl, trifluoromethyl, or β,β,β-trichloroethoxymethyl group; an alkenyl group such as allyl group; an unsubstituted aryl group such as phenyl group; a substituted aryl group having a substituent group such as alkyl, halogen, hydroxyl or alkoxy group, e.g. p-methylphenyl, p-chlorophenyl or p-methoxyphenyl group; an unsubstituted aralkyl group such as benzyl group; a substituted aralkyl group having a substituent group such as alkyl, halogen, hydroxyl or alkoxy group, e.g. p-methylbenzyl, p-chlorobenzyl, p-hydroxybenzyl or p-methoxybenzyl group.

The microorganisms used in the present invention having a stereoselective esterase activity capable of asymmetrically hydrolyzing the compound [(R,S)—II] so as to substantially produce the compound [(R)—I] can be easily selected by the following screening method.

A representative example of the screening method is as follows: In case of using (R,S)-(±)-3-t-butyl-5-acetoxymethyloxazolidin-2-one as a substrate, first, a microorganism is cultured with shaking at a temperature of from 25° to 35°C for 1 to 2 days in a large tube containing 10 ml of a culture medium in which the microorganism can grow. To the resulting cultured broth is added 2% (w/v) of (R,S)-(±)-3-t-butyl-5-acetoxymethyloxazolidin-2-one and the mixture is incubated at a temperature of from 20° to 35°C for 1 to 3 days. During the incubation, a pH of the mixture is kept between 5 and 7, and the amount of the substrate in the reaction mixture is assayed by gas chromatography (column: Silicone OV—17, 1 m × 3 mmφ, Gaschro Kogyo INC.; column temperature: 180°C). And a strain having a hydrolysis rate which is particularly slow down at the time when about 50% of the total amount of the substrate is hydrolyzed is primarily selected.

Further, the culture and hydrolysis are carried out by using the selected strain in the same manner as described above except that the scale of reaction increases by a volume of 400 ml. After completing the reaction, the mixture is extracted with 400 ml of ethyl acetate, and the extract is applied to a column of silica gel and eluted with a mixed solvent of hexane and acetone for separating the unreacted 3-t-butyl-5-acetoxymethyloxazolidin-2-one, and a fraction of 3-t-butyl-5-hydroxymethyloxazolidin-2-one is concentrated. When hexane is slowly added to the concetrate, a crystal is allowed to precipitate. After drying the precipitate under vacuum, it can be easily determined whether or not the used microorganism has a stereoselective esterase activity by measuring a specific optical rotatory power of the precipitate.

An enzyme having a stereoselective esterase activity capable of asymmetrically hydrolyzing the compound [(R,S)—II] can be also selected in the same screening method as in the selection of a microorganism except that a cell-free enzyme extract prepared by centrifuging the homogenized suspension of the microorganism, a partially purified enzyme prepared by fractionating the cell-free enzyme extract or a commercially available enzyme is used instead of a cell suspension.

In the above screening method, the stereoselective esterase activity which is undetectable or hard to detect by using a cell suspension can be detected.

The microorganisms used in the present invention, having a stereoselective esterase activity capable of asymmetrically hydrolyzing the compound [(R,S)—II] so as to substantially produce the compound [(R)—I] are selected from bacteria, yeasts, molds, and the like. Examples of the genera of the microorganisms are, for instance, *Enterobacter, Klebsiella, Micrococcus, Citrobacter, Escherichia, Pseudomonas, Staphylococcus, Alcaligenes, Achromobacter, Streptomyces, Candida, Debaryomyces, Endomyces, Hansenula, Geotrichum, Kluyveromyces, Metschnikowie, Pichia,* and the like. Examples of the strains of the microorganisms are, for instance, *Enterobacter aerogenes* IFO 12010, IFO 13534; *Enterobacter cloacae* IFO 3320, IFO 12935; *Klebsiella pneumoniae* IFO 3318, IFO 3321; *Micrococcus luteus* IFO 3064, IFO 3066; *Citrobacter freundii* IFO 12681; *Escherichia coli* IFO 3366; *Pseudomonas cruciviae* IFO 12047; *Pseudomonas fluorescens* IFO 3081; *Staphylococcus aureus* IFO 3761; *Alcaligenes faecalis* IFO 12669; *AchromoBacter parvulus* IFO 13182; *Streptomyces flavovirens* IFO 3412; *Streptomyces aureus* IFO 3175; *Candida pseudotropicalis* IFO 0432; *Candida Kefyr* IFO 0008; *Debaryomyces hansenii* IFO 0017, IFO 0034; *Endomyces geotrichum* CBS 178.71; *Hansenula anomala* IFO 0144, *Hansenula minuta* IFO 0975; *Geotrichum candidum* CBS 187.67, *Kluyveromyces fragilis* IFO 0288, IFO 0541; *Metschnikowia pulcherrima* IFO 0561; *Pichia farinosa* IFO 0459, *Pichia membranaefaciens* IFO 0186.

For culture of the above microorganisms, any medium in which they can grow can be used. The media usually contain an organic and inorganic carbon source and nitrogen source, and if necessary, vitamins, mineral, and so on. The culture is carried out at a temperature of from 20° to 40°C and a pH of from 3 to 11, preferably from 4 to 8, and if necessary, the culture is aerobically carried out with shaking for stimulating a growth of the microorganism.

For asymmetric hydrolysis reaction of the compound [(R,S)—II], the compound [(R,S)—II] is added to a culture medium at the beginning of the culture so as to simultaneously carry out the culture and the hydrolysis, or the compound [(R,S)—II] is subjected to the action of a cultured broth so as to carry out the hydrolysis as mentioned above. A hydrolysis method, in which the compound [(R,S)—II] is added to a cell suspension with a high concentration obtained by centrifugation, and so on, is preferred in point of recoverying the products after the hydrolysis.

Alternatively, a cell-free enzyme extract prepared by centrifuging the homogenized suspension of the microorganism as described above can be used for the stereoselective hydrolysis of the compound

[(R,S)—II]. A commercially available enzyme prepared from microorganisms can also be used for the stereoselective hydrolysis of the compound [(R,S)—II]. For example, stereoselective L.P.L. "Amano" (lipoprotein lipase from *Pseudomonas,* Amano Pharmaceutical Co., Ltd.), Lipase PL 266 (from *Alcaligenes,* Meito Sangyo Co., Ltd.), or Lipase AL (from *Achromobacter,* Meito Sangyo Co., Ltd.). An immobilized form of the microorganisms or the enzymes ia also available from the stereoselective hydrolysis of the compound [(R,S)—II] instead of the free microorganisms described above.

An enzyme fraction derived from an animal tissue having a stereoselective esterase activity capable of asymmetrically hydrolyzing the compound [(R,S)—II] is also available for the process of the present invention.

A representative screening method is carried out as follows:

To a hog liver is added a phosphate buffer solution in an amount of 4-fold volume of the liver, the mixture is homogenized with a Waring blender under cooling under ice and centrifuged under cooling for 20 minutes at 10,000 × g to obtain a supernatant. To the resulting supernatant is added dropwise ethanol of −20°C to give a suitable concentration, the mixture is allowed to stand for 15 minutes, and then centrifuged for 15 minutes at 10,000 × g under cooling to separate a precipitate. Further, to the resulting supernatent is added dropwise ethanol of −20°C, the mixture is treated in the same manner as described above and centrifuged for 15 minutes at 10,000 × g under cooling to obtain a precipitate and a supernatant. The same procedure as described above is repeated to give crude enzyme fractions which are precipitated in various ethanol concentrations. To a large tube containing 10 ml of phosphate buffer solution of pH 7.0 are added a racemate of 3-isopropyl-5-acetoxymethyloxazolidin-2-one (as a substrate) in an amount of 1% by weight and 0.1 g of the above crude enzyme fraction, and then the mixture is incubated with shaking at 30°C while adjusting a pH of the mixture in the range of 5 to 7. The resulting reaction mixture is treated in the same manner as in the microorganism to give an enzyme fraction having a stereoselective esterase activity capable of asymmetrically hydrolyzing the compound [(R,S)—II].

An enzyme having a stereoselective esterase activity capable of asymmetrically hydrolyzing the compound [(R,S)—II] can be also selected in the same screening method as in the selection of an animal tissue except that a partially purified enzyme fraction prepared by fractionation or a commercially available enzyme is used instead of a crude enzyme.

In the above screening method, the stereoselective esterase activity which is undetectable or hard to detect by using a crude enzyme can be detected.

The available animal tissues are, for instance, bovine, liver, hog liver, rat liver, rabbit liver, chicken liver, bovine pancreas, hog pancreas.

The above fractionation is carried out by a method such as precipitating of the supernatent prepared by centrifugation of the homogenized animal tissue with ethanol, acetone or ammonium sulfate, chromatography of the supernatent by using an ion exchange resin, and the like, or the combination method thereof. More particularly, when the fractionation is carried out by adding dropwise ethanol to the supernatant, the crude enzyme is preferably selected from a fraction being not precipitated in an ethanol concentration of 25% by volume, more preferably a fraction being precipitated in an ethanol concentration of from 27% to 34%. Similarly, in case of fractionating with acetone, the crude enzyme is preferably selected from a fraction being not precipitated in an acetone concentration of 25% by volume, more preferably a fraction being precipitated in an acetone concentration of from 25% to 30%, and in case of fractionating with ammonium sulfate, the crude enzyme is preferably selected from a fraction being not precipitated in 55% by weight saturation with ammonium sulfate, more preferably a fraction being precipitated between 60% and 65% saturation with ammonium sulfate. When the fractionation is carried out by using an ion exchange resin such as DEAE-cellulose, the above crude enzyme is applied to a column of DEAE-cellulose and eluted with a linear gradient of NaCl, and a fraction eluted with a NaCl concentration of from 0.5 M to 0.75 M is collected to obtain the crude enzyme having an activity of asymmetric hydrolysis.

The concentration of the compound [(R,S)—II] in the reaction mixture may be varied from 0.1% by weight to a concentration as high as 30% by weight.

Though the compound [(R,S)—II] has a poor solubility in water, this is no obstacle to the hydrolysis reaction since sufficiently contact of the compound [(R,S)—II] with the enzyme is achieved by shaking. A hydrophilic solvent such as acetone or a surface-active agent may be added to the reaction mixture in an amount which does not affect the reaction.

The hydrolysis reaction is preferably carried out at a pH of from 4 to 8.

When the compound [(R,S)—II] is hydrolyzed in a high concentration, it is preferred to maintain the optimal pH with a suitable neutralizing agent such as a sodium hydroxide solution, since the hydrolyzed organic acid is gradually accumulated in the reaction mixture and the pH of the reaction mixture is converted into acidic side. The hydrolysis reaction is carried out at a temperature suitable to the utilized microorganism, generally at a temperature of from 10° to 50°C.

The separation and recovery of the unreacted compound [(S)—II] from the produced organic acid and the compound [(R)—I] can be carried out by a conventional purification method. An example of the method is as follows: After removing the insoluble materials such as cells from the reaction mixture by centrifugation, the reaction mixture is extracted with an organic solvent usually used, such as hexane, cyclohexane, ether, butyl acetate, chloroform, dichloromethane, benzene or toluene and the extract is concentrated under a reduced pressure to obtain the unreacted compound [(S)—II].

However, the unreacted compound [(S)—II] is not completely separated from thė produced organic acid and the compound [(R)—I] by the above-mentioned solvent extraction method, when the substituent group defined as Y in the formula [(S)—II] is a lower alkyl group such as methyl, ethyl or propyl group. In that case, after removing the insoluble materials in the same manner as described above, the reaction mixture is extracted with an organic solvent such as ethyl acetate and the extract is concentrated under a reduced pressure. The concentrate is subjected to column chromatography to easily separate and recover the compound [(S)—II]. The column chromatography is carried out by using a conventional carrier such as silica gel, alumina or florisil. On the other hand, the compound [(S)—II] also can be easily separated and recovered by a fractional distillation procedure when there is a difference between the reflux temperatures of the compounds [(S)—II] and [(R)—I].

The compound [(S)—II] can be converted into the compound [(S)—I] by the following hydrolysis method. For example, a chemical hydrolysis method in which the compound [(S)—II] is suspended in water and allowed to stand at a room temperature for several hours while adjusting a pH of the mixture in the range of 8 to 14, preferably 12 to 13.5, with a suitable neutralizing agent such as a sodium hydroxide solution, or an enzymatic hydrolysis method in which the compound [(S)—II] is subjected to the action of a non-stereoselective hydrolase, e.g. a lipase (steapsin), an esterase such as one derived from a hog liver, at a temperature of from 20° to 40°C while adjusting a pH of the mixture in the range of 4 to 8. Then, crystals of the compound [(S)—I] can be obtained by extracting the produced compound [(S)—I] with an organic solvent such as ethyl acetate, and concentrating the extract under a reduced pressure.

The compound [(S)—I] can be easily converted into various kinds of β-blockers by conventional methods such as those shown as follows:

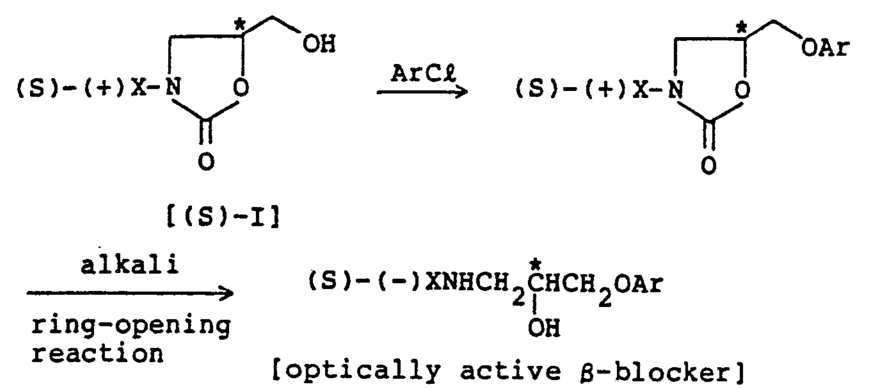


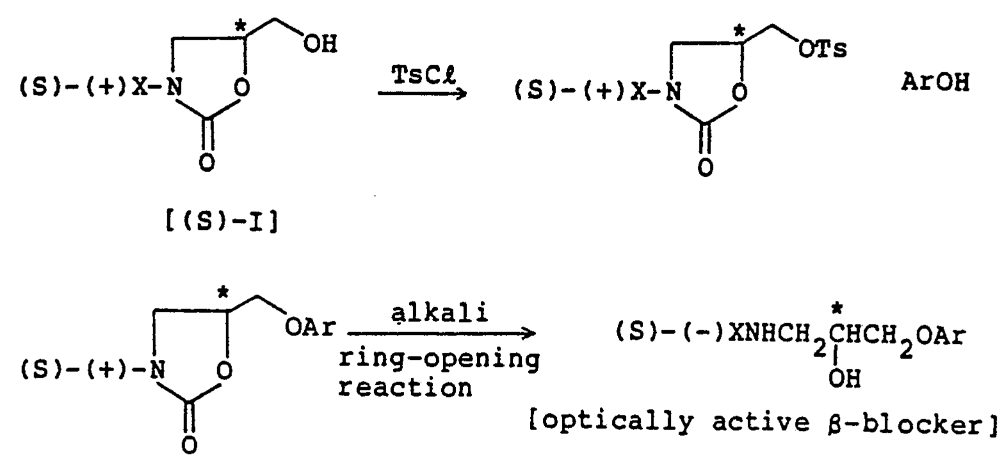


wherein Ar is an aryl derivative, X is as defined above.

The present invention is more particularly described and explained by means of the following Examples, in which all % are by weight unless otherwise noted. These examples are intended to illustrate the invention and not to be construed to limit the scope of the invention.

Also, the microorganisms utilized in the following Examples are all previously known, and strains marked with IFO or CBS are those deposited in the following depositories under the shown catalogue numbers.

IFO: Institute for Fermentation, Osaka (Japan)

CBS: Centraal Bureau voor Schimmelcurtures (Netherlands)

Example 1

The medium of the following components was prepared, and 400 ml portions thereof were separately placed in 2 l of a Sakaguchi flask and steam-sterilized at 120°C for 15 minutes.

[Medium components]

| | |
|---|---|
| Glucose | 4.0 % |
| Yeast extract | 0.3% |
| Meat extract | 0.3% |
| Peptone | 0.3% |
| $(NH_4)_2HPO_4$ | 0.2% |
| $KH_2PO_4$ | 0.1% |
| pH | 7.0 |

On the other hand, *Enterobacter aerogenes* IFO 12010 was previously cultured in the medium containing the same components as described above, and then 10 ml portions thereof were separately inoculated into the above medium and cultured with shaking at 30°C for 24 hours. The cells were collected from 4 liters of the cultured broth by centrifugation and the cells were suspended in 400 ml of 0.1 M phosphate buffer of pH 7.0 and 40 g of (R,S)-(±)-3-t-butyl-5-acetoxymethyloxazolidin-2-one having the formula:

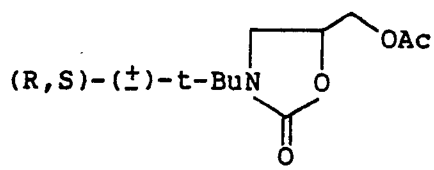

was added to the suspension as a substrate. The thus obtained mixture was incubated in 1 l of a vessel with stirring at 30°C for 18 hours while adjusting a pH of the mixture in the range of 5 to 7 with a sodium hydroxide solution.

After completing the reaction, the resulting supernatant obtained by centrifugation was extracted twice with each 400 ml of ethyl acetate, and then the extract was concentrated under reduced pressure.

The obtained concentrate was chromatographed on a column of silica gel, and the fractions of (S)-(+)-3-t-butyl-5-acetoxymethyloxazolidin-2-one eluted with a mixed solvent of hexane-acetone (3:1 by v/v) were gathered and concentrated under a reduced pressure. After removing the solvent, 18.5 g of colorless oily material was obtained. The value of a specific optical rotatory power is $[\alpha]_D^{16}$ +36.3° (C = 1.0, chloroform), and the NMR spectrum is as follows:

$^1$H-NMR (90 MHz, in $CDCl_3$) δ (ppm): 1.4 (9H, s, $C(CH_3)_3$, 2.1 (3H, s,

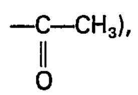

3.3—3.8 (2H, m, —$CH_2N$—), 4.1—4.25 (2H, m, —$CH_2O$—), 4.4—4.7 (1H, m, —$CH_2$*CH*(O—)$CH_2$).

The thus obtained (S)-(+)-3-t-butyl-5-acetoxymethyloxazolidin-2-one was suspended in 100 ml of water, and the resulting mixture was hydrolyzed at a room temperature for two days while adjusting the pH of the mixture in the range of 10 to 12 with a sodium hydroxide solution.

After completing the hydrolysis, the mixture was extracted with 200 ml of ethyl acetate, and the resulting extract was dehydrated, and then concentrated under a reduced pressure. When hexane was slowly added to the concentrate, colorless crystals precipitated. The precipitated crystals were dried under vacuum to give 12.9 g of (S)(+)-3-t-butyl-5-hydroxymethyloxazolidin-2-one having a specific optical rotatory power of $[\alpha]_D^{16}$ +46.0° (C = 1.0, chloroform).

The result of NMR spectrum is as follows: $^1$H-NMR (90 MHz,in $CDCl_3$) δ (ppm): 1.4 (9H, s, $C(CH_3)_3$), 3.4—3.95 (5H, —$CH_2N$—, —$CH_2O$—, —OH), 4.3—4.6 (1H, m, —$CH_2$*CH*(O—)$CH_2$—).

Examples 2 to 14

The procedure of Example 1 was repeated except that the strain of microorganism was changed as shown in Table 1. The results are shown in Table 1.

Table 1

Substrate: (R,S)-(±)-3-t-butyl-5-acetoxymethyloxazolidin-2-one

| Example | Strain | | (S)-(+)-3-t-butyl-5-acetoxymethyloxazolidin-2-one | | (S)-(+)-3-t-butyl-5-hydroxymethyloxazolidin-2-one | |
|---|---|---|---|---|---|---|
| | | | Yield g | $[\alpha]_D^{16}$ (C=1.0, chloroform) | Yield g | $[\alpha]_D^{16}$ (C=1.0, chloroform) |
| 2 | *Enterobacter aerogenes* | IFO 13534 | 17.8 | +36.4° | 12.5 | +46.2° |
| 3 | *Enterobacter cloacae* | IFO 3320 | 16.8 | +36.1° | 11.8 | +45.8° |
| 4 | " | IFO 12935 | 16.5 | +35.9° | 11.4 | +45.6° |
| 5 | *Klebsiella pneumoniae* | IFO 3318 | 17.2 | +37.0° | 11.9 | +47.0° |
| 6 | " | IFO 3321 | 17.6 | +36.8° | 12.2 | +46.8° |
| 7 | *Micrococcus luteus* | IFO 3064 | 16.9 | +33.2° | 11.8 | +42.5° |
| 8 | " | IFO 3066 | 17.1 | +32.9° | 11.9 | +41.8° |
| 9 | *Escherichia coli* | IFO 3366 | 17.0 | +28.7° | 11.7 | +36.5° |
| 10 | *Pseudomonas cruciviae* | IFO 12047 | 16.8 | +29.5° | 11.7 | +37.8° |
| 11 | *Pseudomonas fluorescens* | IFO 3081 | 17.0 | +30.1° | 11.9 | +38.1° |
| 12 | *Staphylococcus aureus* | IFO 3761 | 16.9 | +25.6° | 11.8 | +32.5° |
| 13 | *Alcaligenes faecalis* | IFO 12669 | 17.1 | +21.9° | 12.0 | +28.1° |
| 14 | *Achromobacter parvulus* | IFO 13182 | 17.2 | +18.4° | 11.8 | +23.4° |

Examples 15 to 28

The procedure of Example 1 was repeated except that the strain of microorganism was changed as shown in Table 2 and (R,S)-(±)-3-isopropyl-5-acetoxymethyloxazolidin-2-one was employed as a substrate. The results are shown in Table 2.

The result of NMR spectrum of (S)-(+)-3-isopropyl-5-hydroxymethyloxazolidin-2-one is as follows:

$^1$H-NMR (90 MHz, in $CDCl_3$) δ (ppm): 1.2 (6H, d, —CH($CH_3$)$_2$), 3.4—4.2 (6H, m, —$CH_2$N—, —$CH_2$O— ($CH_3$)$_2$CH—, —OH), 4.3—4.7 (1H, m, —$CH_2$CH(O—)$CH_2$—)

Table 2

Substrate: (R,S)-(±)-3-isopropyl-5-acetoxymethyloxazolidin-2-one

| Example | Strain | | (S)-(+)-3-isopropyl-5-acetoxymethyloxazolidin-2-one | | (S)-(+)-3-isopropyl-5-hydroxymethyloxazolidin-2-one | |
|---|---|---|---|---|---|---|
| | | | Yield g | $[\alpha]_D^{20}$ (C=1.0, chloroform) | Yield g | $[\alpha]_D^{20}$ (C=1.0, chloroform) |
| 15 | *Enterobacter aerogenes* | IFO 12010 | 17.7 | +42.3° | 11.9 | +54.2° |
| 16 | " | IFO 13534 | 17.1 | +42.7° | 11.5 | +54.7° |
| 17 | *Enterobacter cloacae* | IFO 3320 | 16.9 | +42.9° | 11.3 | +54.9° |
| 18 | " | IFO 12935 | 17.0 | +43.0° | 11.4 | +55.0° |
| 19 | *Klebsiella pneumoniae* | IFO 3318 | 17.1 | +43.3° | 11.5 | +55.4° |
| 20 | " | IFO 3321 | 17.2 | +43.0° | 11.6 | +55.1° |
| 21 | *Micrococcus luteus* | IFO 3064 | 16.8 | +39.3° | 11.3 | +50.0° |
| 22 | " | IFO 3066 | 16.5 | +38.9° | 11.1 | +49.8° |
| 23 | *Escherichia coli* | IFO 3366 | 17.0 | +33.4° | 11.5 | +42.8° |
| 24 | *Pseudomonas cruciviae* | IFO 12047 | 17.1 | +32.8° | 11.2 | +41.9° |
| 25 | *Pseudomonas fluorescens* | IFO 3081 | 17.5 | +33.7° | 11.8 | +43.1° |
| 26 | *Staphylococcus aureus* | IFO 3761 | 16.6 | +30.1° | 11.0 | +38.2° |
| 27 | *Alcarigenes faecalis* | IFO 12669 | 17.3 | +24.9° | 11.4 | +31.9° |
| 28 | *Achromobacter parvulus* | IFO 13182 | 17.0 | +21.0° | 10.9 | +27.0° |

Example 29

The medium of the following components was prepared, and 400 ml portions thereof were separately placed in 2 l of a Sakaguchi flask and steam-sterilized at 120°C for 15 minutes.

[Medium components]

| | |
|---|---|
| Glucose | 2.0% |
| Yeast extract | 0.3% |
| $(NH_4)_2HPO_4$ | 1.3% |
| $KH_2PO_4$ | 0.7% |
| NaCl | 0.1% |
| $ZnSO_4 \cdot 7H_2O$ | 0.006% |
| $FeSO_4 \cdot 7H_2O$ | 0.009% |
| pH | 6.5 |

On the other hand, *Candida pseudotropicalis* IFO 0432 was previously cultured in the medium containing the same components as described above, and then 10 ml portions thereof were separately inoculated into the above medium and cultured with shaking at 30°C for 24 hours. The cells were collected from 4 liters of the cultured broth by centrifugation and the cells were suspended in 400 ml of 0.1 M phosphate buffer of pH 7.0, and 40 g of (R,S)-(±)-3-t-butyl-5-acetoxymethyloxazolidin-2-one having the formula:

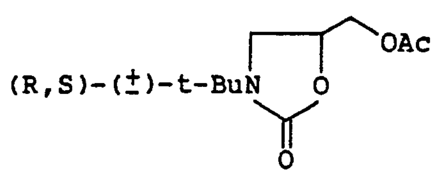

was added to the mixture as a substrate. The thus obtained mixture was subjected to reaction in 1 l of a vessel with stirring at 30°C for 18 hours while adjusting a pH of the mixture in the range of 5 to 7 with a sodium hydroxide solution. After completing the reaction, the supernatant obtained by centrifugation was extracted twice with each 400 ml of ethyl acetate, and then the extract was concentrated under a reduced pressure.

The thus obtained concentrate was chromatographed on a column gel, and the fraction of (S)-(+)-3-t-butyl-5-acetoxymethyloxazolidin-2-one eluted with a mixed solvent of hexane-acetone (3:1, v/v) were gathered and concentrated under a reduced pressure. After removing the solvent, 17.5 g of colorless oily material was obtained. The value of a specific optical rotatory power is $[\alpha]_D^{16}$ +24.5° (C = 1.0, chloroform). The thus obtained (S)-(+)-3-t-butyl-5-acetoxymethyloxazolidin-2-one was suspended in 100 ml of water, and the resulting mixture was hydrolyzed at a room temperature for two days while adjusting the pH of the mixture in the range of 10 to 12 with a sodium hydroxide solution.

After completing the hydrolysis, the mixture was extracted with 200 ml of ethyl acetate, and the extract was dehydrated, and then concentrated under a reduced pressure. When hexane was added to the concentrate, colorless crystals precipitated. The precipitated crystals were gathered and dried under vacuum to give 11.5 g of (S)-(+)-3-t-butyl-5-hydroxymethyloxazolidin-2-one having a specific optical rotatory power of $[\alpha]_D^{16}$ +31.1° (C = 1.0, chloroform).

Examples 30 to 41

The procedure of Example 29 was repeated except that the strain of microorganism was changed as shown in Table 3. The results are shown in Table 3.

Table 3

Substrate: (R,S)-(±)-3-t-butyl-5-acetoxymethyloxazolidin-2-one

| Example | Strain | | (S)-(+)-3-t-butyl-5-acetoxymethyloxazolidin-2-one | | (S)-(+)-3-t-butyl-5-hydroxymethyloxazolidin-2-one | |
|---|---|---|---|---|---|---|
| | | | Yield g | $[\alpha]_D^{16}$ | Yield g | $[\alpha]_D^{16}$ |
| | | | | (C=1.0, chloroform) | | (C=1.0, chloroform) |
| 30 | *Candida kefyr* | IFO 0008 | 16.2 | +24.5° | 10.5 | +31.0° |
| 31 | *Debaryomyces hansenii* | IFO 0017 | 17.1 | +30.0° | 11.1 | +38.2° |
| 32 | " | IFO 0034 | 16.3 | +29.3° | 10.6 | +37.5° |
| 33 | *Endomyces geotrichum* | CBS 178.71 | 15.8 | +16.5° | 9.9 | +20.9° |
| 34 | *Hansenula anomala* | IFO 0144 | 16.0 | +14.9° | 10.0 | +18.8° |
| 35 | *Hansenula minuta* | IFO 0975 | 16.2 | +15.0° | 10.2 | +19.1° |
| 36 | *Geotrichum candidum* | CBS 187.67 | 16.5 | +13.5° | 10.7 | +17.2° |
| 37 | *Kluyveromyces fragilis* | IFO 0288 | 15.5 | +17.7° | 9.9 | +22.5° |
| 38 | " | IFO 0541 | 15.9 | +17.2° | 10.0 | +21.9° |
| 39 | *Metschnikowia pulcherrima* | IFO 0561 | 16.4 | +24.1° | 10.5 | +30.8° |
| 40 | *Pichia farinosa* | IFO 0459 | 17.0 | +26.4° | 11.0 | +33.5° |
| 41 | *Pichia membranaefaciens* | IFO 0186 | 15.9 | +25.4° | 10.1 | +32.2° |

Examples 42 to 54

The procedure of Example 29 was repeated except that the strain of microorganism was changed as shown in Table 4 and (R,S)-(±)-3-isopropyl-5-acetoxymethyloxazolidin-2-one was employed as a substrate. The results are shown in Table 4.

**Table 4**

Substrate: (R,S)-(±)-3-isopropyl-5-acetoxymethyloxazolidin-2-one

| Example | Strain | | (S)-(+)-3-isopropyl-5-acetoxymethyloxazolidin-2-one | | (S)-(+)-3-isopropyl-5-hydroxymethyloxazolidin-2-one | |
|---|---|---|---|---|---|---|
| | | | Yield g | $[\alpha]_D^{20}$ | Yield g | $[\alpha]_D^{20}$ |
| | | | | (C=1.0, chloroform) | | (C=1.0, chloroform) |
| 42 | *Candida pseudotropicalis* | IFO 0432 | 17.2 | +29.0° | 11.1 | +37.1° |
| 43 | *Candia kefyr* | IFO 0008 | 15.5 | +29.1° | 10.0 | +37.2° |
| 44 | *Debaryomyces hansenii* | IFO 0017 | 16.0 | +35.7° | 10.3 | +45.7° |
| 45 | " | IFO 0034 | 16.2 | +35.4° | 10.5 | +44.9° |
| 46 | *Endomyces geotrichum* | CBS 178.71 | 15.4 | +19.7° | 9.4 | +25.0° |
| 47 | *Hansenula anomala* | IFO 0144 | 15.6 | +17.4° | 9.8 | +22.1° |
| 48 | *Hansenula minuta* | IFO 0975 | 15.8 | +18.0° | 9.7 | +22.9° |
| 49 | *Geotrichum candidum* | CBS 187.67 | 15.1 | +16.1° | 9.2 | +20.5° |
| 50 | *Kluyveromyces fragilis* | IFO 0288 | 14.8 | +21.3° | 9.6 | +27.0° |
| 51 | " | IFO 0541 | 14.2 | +20.5° | 9.2 | +26.3° |
| 52 | *Metschnikowia pulcherrima* | IFO 0561 | 15.6 | +29.1° | 9.3 | +36.9° |
| 53 | *Pichia farinosa* | IFO 0459 | 15.9 | +31.5° | 9.9 | +40.0° |
| 54 | *Pichia membranaefaciens* | IFO 0186 | 16.2 | +30.5° | 10.2 | +38.5° |

Example 55

The medium of the following components was prepared, and 400 ml portions thereof were separately placed in 2 l of a Sakaguchi flask and steam-sterilized at 120°C for 15 minutes.

[Medium components]

| | |
|---|---|
| Glucose | 2.0% |
| Glycerol | 2.0% |
| Soybean meal | 3.0% |
| Yeast extract | 0.3% |
| Vitamin $B_{12}$ | 1 ppm |
| pH | 7.2 |

On the other hand, *Streptomyces flavovirens* IFO 3412 was previously cultured in the medium containing the same components as described above, and then 10 ml portions thereof were separately inoculated into the above medium and cultured with shaking at 30°C for 48 hours.

The cells were collected from 4 liters of the cultured broth by centrifugation and the cells were suspended in 400 ml of 0.1 M phosphate buffer of pH 7.0, and 40 g of (R,S)-(±)-3-t-butyl-5-acetoxymethyloxazolidin-2-one having the formula:

(R,S)-(±)-t-Bu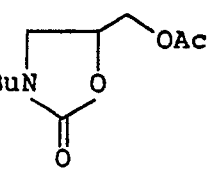

was added to the suspension as a substrate. The thus obtained mixture was subjected to reaction in 1 l of a vessel with stirring at 30°C for 18 hours while adjusting pH of the mixture in the range of 5 to 7 with a sodium hydroxide solution. After completing the reaction, the supernatant obtained by centrifugation was extracted twice each time with 400 ml of ethyl acetate, and then the extract was concentrated under a reduced pressure.

The obtained concentrate was chromatographed on a column of silica gel, and the fractions of (S)-(+)-3-t-butyl-5-acetoxymethyloxazolidin-2-one eluted with a mixed solvent of hexane-acetone (3:1 v/v) were gathered and concentrated under a reduced pressure. After removing the solvent, 16.9 g of colorless oily material was obtained. The value of a specific optical rotatory power is $[\alpha]_D^{16}$ + 22.6° (C = 1.0, chloroform).

The thus obtained (S)-(+)-3-t-butyl-5-acetoxymethyloxazolidin-2-one was suspended in 100 ml of water, and the resulting mixture was hydrolyzed at a room temperature for two days while adjusting the pH of the mixture in the range of 10 to 12 with a sodium hydroxide solution.

After completing the hydrolysis, the mixture was extracted with 200 ml of ethyl acetate, and the extract was dehydrated, and then concentrated under a reduced pressure. When hexane was slowly added to the concentrate, colorless crystals precipitated. The precipitated crystals were gathered and dried under vacuum to give 11.1 g of (S)-(+)-3-t-butyl-5-hydroxymethyloxazolidin-2-one having specific optical rotatory power of $[\alpha]_D^{16}$ +28.7 (C = 1.0, chloroform).

Example 56

The procedure of Example 51 was repeated except that the strain of microorganism was changed as shown in Table 5. The results are shown in Table 5.

Table 5

Substrate: (R,S)-($\pm$)-3-t-butyl-5-acetoxymethyloxazolidin-2-one

| Example | Strain | (S)-(+)-3-t-butyl-5-acetoxymethyloxazolidin-2-one | | (S)-(+)-3-t-butyl-5-hydroxymethyloxazolidin-2-one | |
|---|---|---|---|---|---|
| | | Yield g | $[\alpha]_D^{16}$ (C=1.0, chloroform) | Yield g | $[\alpha]_D^{16}$ (C=1.0, chloroform) |
| 56 | *Staphylococcus aureus* IFO 3175 | 16.8 | $+16.0^{\circ}$ | 11.0 | $+21.0^{\circ}$ |

Examples 57 to 58

The procedure of Example 55 was repeated except that the strain of microorganism was changed as shown in Table 6 and (R,S)-(±)-3-isopropyl-5-acetoxymethyloxazolidin-2-one was employed as a substrate. The results are shown in Table 6.

Table 6

Substrate: (R,S)-(±)-3-isopropyl-5-acetoxymethyloxazolidin-2-one

| Example | Strain | | (S)-(+)-3-isopropyl-5-acetoxymethyloxazolidin-2-one | | (S)-(+)-3-isopropyl-5-hydroxymethyloxazolidin-2-one | |
|---|---|---|---|---|---|---|
| | | | Yield g | $[\alpha]_D^{20}$ (C=1.0, chloroform) | Yield g | $[\alpha]_D^{20}$ (C=1.0, chloroform) |
| 57 | *Streptomyces flavovirens* | IFO 3412 | 16.6 | +26.9° | 9.9 | +34.4° |
| 58 | *Streptomyces aureus* | IFO 3175 | 16.2 | +19.6° | 9.8 | +25.1° |

Example 59

The medium of the following components was prepared, and 400 ml portions thereof were separately placed in a 2 l of a Sakaguchi flask and steam-sterilized at 120°C for 15 minutes.

[Medium components]

| | |
|---|---|
| Glucose | 4.0% |
| Yeast extract | 0.3% |
| Meat extract | 0.3% |
| Peptone | 0.3% |
| $(NH_4)_2HPO_4$ | 0.2% |
| $KH_2PO_4$ | 0.1% |
| pH | 7.0 |

On the other hand, *Enterobacter aerogenes* IFO 12010 had been previously cultured in the medium containing the same components as described above, and then 10 ml portions thereof were separately inoculated into the above medium and cultured with shaking at 30°C for 24 hours. The cells were collected from 4 liters of the cultured broth by centrifugation and the cells were suspended in 400 ml of 0.1 M phosphate buffer of pH 7.0, and 40 g of (R,S)-(±)-3-t-butyl-5-benzoyloxymethyloxazolidin-2-one having the formula:

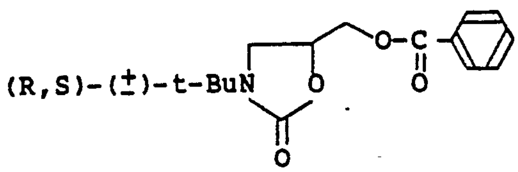

was added to the suspension as a substrate. The thus obtained mixture was subjected to reaction with stirring at 30°C for 40 hours while adjusting the pH of the mixture in the range of 5 to 7 with a sodium hydroxide solution. After completing the reaction, the supernatant obtained by centrifugation was extracted with 400 ml of toluene to give ureacted (S)-(+)-3-t-butyl-5-benzoyloxymethyloxazolidin-2-one, and the resulting extract was concentrated under a reduced pressure.

Into 200 ml of 0.1 M phosphate buffer was suspended the obtained concentrate and added 2 g of lipase (steapsin). The mixture was hydrolyzed with stirring at 30°C for 18 hours while adjusting a pH of the mixture in the range of 5 to 7 with a sodium hyroxide solution.

After completing the hydrolysis, the mixture was extracted with 400 ml of ethyl acetate, and the extract was dehydrated, and then concentrated under a reduced pressure. When hexane was added to the concentrate, colorless crystals precipitated. The precipitated crystals were gathered and dried under vacuum to give 12.8 g of (S)-(+)-3-t-butyl-5-hydoxymethyloxazolidin-2-one having a specific optical rotatory power of $[\alpha]_D^{16}$ +46.1° (C = 1.0, chloroform).

Examples 60 to 74

The procedure of Example 59 was repeated except that the strain of microorganism and the substrate were changed as shown in Table 7, respectively. The results are shown in Table 7.

Table 7

| Example | Strain | | Substrate (R,S)-(±)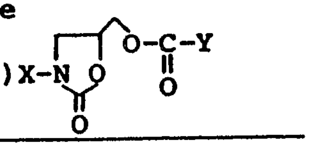 X | Y | Product (S)-(+)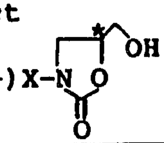 Yield g | $[\alpha]_D^{**}$ (C=1.0, chloroform) |
|---|---|---|---|---|---|---|
| 60 | *Enterobacter aerogenes* | IFO 12010 | t-butyl | trifluoromethyl | 10.9 | $+45.1^o$ |
| 61 | " | | " | dichloromethyl | 11.2 | $+45.6^o$ |
| 62 | " | | " | benzyl | 11.5 | $+45.9^o$ |
| 63 | " | | isopropyl | phenyl | 11.6 | $+54.2^o$ |
| 64 | " | | " | trifluoromethyl | 10.5 | $+53.5^o$ |
| 65 | " | | " | dichloromethyl | 11.0 | $+53.9^o$ |
| 66 | " | | " | benzyl | 11.1 | $+54.1^o$ |
| 67 | *Klebsiella pneumoniae* | IFO 3318 | t-butyl | phenyl | 12.0 | $+45.2^o$ |
| 68 | " | | " | trifluoromethyl | 11.0 | $+45.0^o$ |
| 69 | " | | " | dichloromethyl | 11.2 | $+45.2^o$ |
| 70 | " | | " | benzyl | 11.4 | $+45.7^o$ |
| 71 | " | | isopropyl | phenyl | 11.7 | $+54.5^o$ |
| 72 | " | | " | trifluoromethyl | 11.0 | $+53.9^o$ |
| 73 | " | | " | dichloromethyl | 9.8 | $+53.2^o$ |
| 74 | " | | " | benzyl | 10.1 | $+54.0^o$ |

** When X is t-butyl, a specific optical rotatory power was measured at $16^oC$, and when X is isopropyl, at $20^oC$.

Example 75

*Enterobacter aerogenes* IFO 12010 was cultured in the same manner as in Example 1 to give 400 ml of cultured broth. To the culture broth was added 40 g of (R,S)-(±)-3-t-butyl-5-acetoxymethyloxazolidin-2-one having the formula:

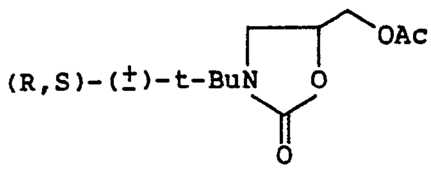

as a substrate. The resulting mixture was subjected to reaction in 1 l of a vessel with aerobically stirring at 30°C for 18 hours while adjusting the pH of the mixture in the range of 5 to 7 with a sodium hydroxide solution. After completing the reaction, the reaction mixture was treated in the same manner as in Example 1 to give 1.03 g of (S)-(+)-3-t-butyl-5-hydroxymethyloxazolidin-2-one having a specific optical rotatory power of $[\alpha]_D^{16}$ +43.9° (C = 1.0, chloroform).

Examples 76 to 78

The procedure of Example 75 was repeated except that the strain of microorganism and the substrate were changed as shown in Table 8, respectively. The results are shown in Table 8.

Table 8

| Example | Strain | | Substrate (R,S)-(±)X-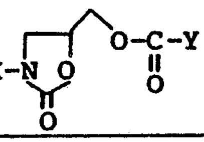 X | Y | Product (S)-(+)X-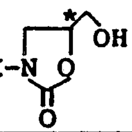 Yield g | $[\alpha]_D^{**}$ (C=1.0, chloroform) |
|---|---|---|---|---|---|---|
| 76 | *Enterobacter aerogenes* | IFO 12010 | isopropyl | methyl | 1.00 | +52.5° |
| 77 | *Klebsiella pneumoniae* | IFO 3318 | t-butyl | methyl | 0.99 | +44.5° |
| 78 | " | | isopropyl | methyl | 0.96 | +52.9° |

** the same as shown in Table 7

Table 9

| Example | Strain | | Substrate (R,S)-(±)X-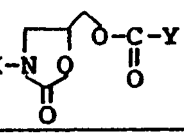 X | Y | Product (S)-(+)X-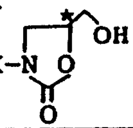 Yield g | $[\alpha]_D^{**}$ (C=1.0, chloroform) |
|---|---|---|---|---|---|---|
| 80 | *Enterobacter aerogenes* | IFO 12010 | isopropyl | methyl | 0.90 | +48.6° |
| 81 | *Klebsiella pneumoniae* | IFO 3318 | t-butyl | methyl | 0.75 | +41.2° |
| 82 | " | | isopropyl | methyl | 0.81 | +47.9° |

** the same as shown in Table 7

Example 79

*Enterobacter aerogenes* IFO 12010 was incoculated into 10 ml of a culture medium containing 7% glucose, 0.3% yeast extract, 0.3% meat extract, 0.3% peptone, 0.2% $(NH_4)_2HPO_4$, 0.1% $KH_2PO_4$ of pH 7.0, and they were cultured with shaking at 30°C for 24 hours.

Into 400 ml of the above culture medium was inoculated the above cultured broth and added simultaneously 4.0 g of (R,S)-(±)-3-t-butyl-5-acetoxymethyloxazolidine-2-one having the formula:

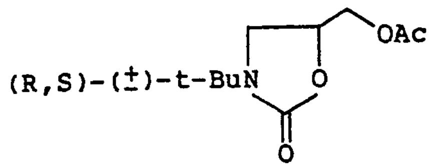

as a substrate, and the culture was carried out at 30°C for 48 hours with shaking.

After completing the reaction, the reaction mixture was treated in the same manner as in Example 1 to give 0.82 g of (S)-(+)-3-t-butyl-5-hydroxymethyloxazolidin-2-one having a specific optical rotatory power of $[\alpha]_D^{16}$ +41.5° (C = 1.0, chloroform).

Examples 80 to 82

The procedure of Example 79 was repeated except that the strain of microorganism and the substrate were changed as shown in Table 9, respectively. The results are shown in Table 9.

Example 83

*Enterobacter aerogenes* IFO 12010 was cultured in the same manner as in Example 1 to give 4 l of culture broth and the cultured broth was subjected to centrifugation to collect cells. The cells were suspended in 400 ml of 0.1 M phosphate buffer of pH 7.0, homogenized with a Broun homogenizer under cooling, and then centrifuged to give a cell-free enzyme extract. To the resulting cell-free enzyme extract was added 4.0 g of (R,S)-(±)-3-t-butyl-5-acetoxymethyloxazolidin-2-one as a substrate, and the mixture was subjected to reaction with stirring at 30°C for 18 hours while adjusting the pH of the mixture in the range of 5 to 7 with a sodium hydroxide solution. The reaction mixture was concentrated under reduced pressure, the resulting concentrate was applied to a column of silica gel, and then treated in the same manner as in Example 1 to give 1.07 g of (S)-(+)-3-t-butyl-5-hydroxymethyloxazolidin-2-one having a specific optical rotatory power of $[\alpha]_D^{16}$ +46.3° (C = 1.0, chloroform).

Examples 84 to 94

The procedure of Example 83 was repeated except that the strain of microorganism and the substrate were changed as shown in Table 10, respectively. The results are shown in Table 10.

Table 10

| Example | Strain | | Substrate (R,S)-(±)X- 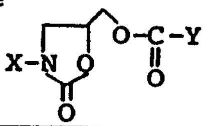 | | Product (S)-(+)X- 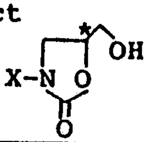 | |
|---|---|---|---|---|---|---|
| | | | X | Y | Yield g | $[\alpha]_D^{**}$ (C=1.0, chloroform) |
| 84 | *Enterobacter aerogenes* | IFO 12010 | isopropyl | methyl | 0.98 | +54.1° |
| 85 | *Klebsiella pneumoniae* | IFO 3318 | t-butyl | methyl | 1.01 | +46.0° |
| 86 | " | | isopropyl | methyl | 1.04 | +54.2° |
| 87 | *Micrococcus luteus* | IFO 3064 | t-butyl | methyl | 1.10 | +41.8° |
| 88 | " | | isopropyl | methyl | 0.95 | +49.4° |
| 89 | *Pseudomonas cruciviae* | IFO 12047 | t-butyl | methyl | 1.00 | +37.3° |
| 90 | " | | isopropyl | methyl | 1.05 | +41.7° |
| 91 | *Alcaligenes faecalis* | IFO 12669 | t-butyl | methyl | 1.12 | +43.5° |
| 92 | " | | isopropyl | methyl | 1.03 | +48.8° |
| 93 | *Achromobacter parvulus* | IFO 13182 | t-butyl | methyl | 1.09 | +24.0° |
| 94 | " | | isopropyl | methyl | 1.08 | +27.4° |

** the same as shown in Table 7

Example 95

One gram of L.P.L.-Amano-3 (lipoprotein lipase: EC. 3.1.1.4., from Pseudomans; Amano Pharmaceutical Co., Ltd.) and 4.0 g of (R,S)-(±)-3-t-butyl-5-acetoxymethyloxazolidin-2-one were added to 40 ml of 0.1 M phosphate buffer of pH 7.0, and the mixture was incubated with stirring at 30°C for 18 hours while adjusting a pH of the mixture in the range of 5 to 7 with a sodium hydroxide solution. After completing the hydrolysis, the reaction mixture was treated in the same manner as in Example 1 to give 1.1 g of (S)-(+)-3-t-butyl-5-hydroxymethyloxazolidin-2-one having a specific optical rotatory power of $[\alpha]_D^{16}$ +42.8° (C = 1.0, chloroform).

Examples 96 to 100

The procedure of Example 95 was repeated except that (R,S)-(±)-3-isopropyl-5-acetoxymethyloxazolidin-2-one was employed as a substrate. The results are shown in Table II.

Table 11

| Example | Enzyme | (Origin) | Substrate (R,S)-($\pm$)X- 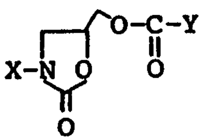 X | Y |
|---|---|---|---|---|
| 96 | L. P. L-Amano-3 (Amano Pharmaceutical Co., Ltd.) | *(Pseudomonas)* | isopropyl | methyl |
| 97 | Lipase PL 266 (Meito Sangyo Co., Ltd.) | *(Alcaligenes)* | t-butyl | methyl |
| 98 | | | isopropyl | methyl |
| 99 | Lipase AL (Meito Sangyo Co., Ltd.) | *(Achromobacter)* | t-butyl | methyl |
| 100 | | | isopropyl | methyl |

- continued -

- continued -

| Example | Product | | | |
|---|---|---|---|---|
| | (S)-(+)X- 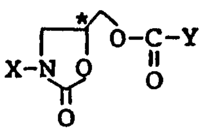 | | (S)-(+)X- 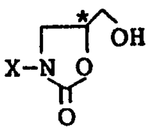 | |
| | Yield g | $[\alpha]_D^{*}$ (C=1.0, chloroform) | Yield g | $[\alpha]_D^{**}$ (C=1.0, chloroform) |
| 96 | 1.67 | +40.1° | 1.12 | +51.1° |
| 97 | 1.62 | +34.9° | 1.01 | +44.5° |
| 98 | 1.64 | +41.2° | 1.09 | +52.4° |
| 99 | 1.70 | +18.8° | 1.10 | +23.9° |
| 100 | 1.16 | +21.1° | 1.04 | +27.3° |

** the same as shown in Table 7

Example 101

Twenty five g of hog liver was added to 100 ml of 0.1 M phosphate buffer of pH 7.0, and the mixture was homogenized with a Waring blender for one minute under cooling. The resulting homogenate was centrifuged for 20 minutes at 10,000 × g. To 100 ml of the supernatant was added dropwise 37 methanol of −20°C (27%, v/v). After standing for 15 minutes, the mixture was centrifuged for 15 minutes at 10,000 × g under cooling. To 100 ml of the resulting supernatant was added dropwise 10.6 ml of ethanol of −20°C (final 34%, v/v). After standing for 15 minutes, the mixture was centrifuged for 15 minutes at 10,000 × g under cooling. Further, the precipitate was dissolved in 100 ml of 0.1 M phosphate buffer of pH 7.0, and ammonium sulfate was added thereto to give a crude enzyme being precipitated between 60% and 65% saturation with ammonium sulfate. Into 100 ml of 0.1 M phosphate buffer of pH 7.0 was dissolved 1 g of the thus obtained crude enzyme and added 10 g of (R,S)-(±)-3-isopropyl-5-acetoxymethyloxazolidin-2-one as a substrate. The hydrolysis was carried out in 500 ml of a vessel with stirring at 30°C for 24 hours while adjusting the pH of the mixture in the range of 5 to 7. After completing the reaction, the reaction mixture was filtered, and the filtrate was extracted three times with each 100 ml of ethyl acetate, and then the extract was concentrated under a reduced pressure. The resulting concentrate was chromatographed on a column of silica gel, and the fractions of (S)-(±)-3-isopropyl-5-acetoxymethyloxazolidin-2-one eluted with a mixed solvent of hexane-acetone (3:1, v/v) were gathered and concentrated under a reduced pressure. After removing the solvent, 4.1 g of colorless oily material was obtained. The value of a specific optical rotatory power is $[\alpha]_D^{20}$ +41.1°C (C = 1.0, chloroform), and the NMR spectrum is as follows:

$^1$H—NMR (90 MHz, in $CDCl_3$) (ppm): 1.2 (6H, d, —$CH(CH_3)_2$), 2.1 (3H, s,

—C—$CH_3$)
‖
O

3.2—4.3 (5H, m, —$CH_2N$—, $(CH_3)_2CH$—, —$CH_2O$), 4.5—4.9 (1H, m, —$CH_2CH(O—)CH_2$—)

The thus obtained (S)-(+)-3-isopropyl-5-acetoxymethyloxazolidin-2-one was suspended in 50 ml of water, and then hydrolyzed at a room temperature for two days while adjusting the pH of the mixture in the range of 10 to 12 with a sodium hydroxide solution.

After completing the hydrolysis, the mixture was extracted with 100 ml of ethyl acetate, and the extract was concentrated after dehydration. When hexane was slowly added to the concentrate, colorless crystals precipitated. The precipitated crystals were dried under vacuum to give 3.0 g of (S)-(+)-3-isopropyl-5-hydroxymethyloxazolidin-2-one having a specific optical rotatory power of $[\alpha]_D^{20}$ +46.3°C (C = 1.0, chloroform). The NMR spectrum is as follows:

$^1$H—NMR (90 MHz, in $CDCl_3$) δ 1.2 (ppm): (6H, d, —$CH(CH_3)_2$), 3.4—4.2 (6H, m, —$CH_2N$—, —$CH_2O$—, $(CH_3)_2CH$—, —OH), 4.3—4.7 (1H, m, —$CH_2CH(O—)CH_2$–)

Example 102

As a crude enzyme was used the fraction obtained from the hog liver homogenate in Example 101 which was precipitated in an ethanol concentration of 27% to 34% (v/v), and (R,S)-(±)-3-isopropyl-5-acetoxymethyloxazolidin-2-one as a substrate was subjected to asymmetric hydrolysis in the same manner as in Example 101. As a result, 4.2 g of (S)-(+)-3-isopropyl-5-acetoxymethyloxazolidin-2-one having a specific optical rotatory power of $[\alpha]_D^{20}$ + 35.6° (C = 1.0, chloroform) was obtained.

The above (S)-(+)-3-isopropyl-5-acetoxymethyloxazolidin-2-one was further hydrolyzed to give 3.0 g of (S)-(+)3-isopropyl-5-hydroxymethyloxazolidin-2-one having a specific optical rotatory power of $[\alpha]_D^{20}$ +40.4° (C =1.0, chloroform).

Example 103

The procedure of Example 101 was repeated except that acetone of −20°C was used instead of ethanol and a fraction being precipitated in an acetone concentration of 25% to 30% was used as a crude enzyme extract to give 3.7 g of (S)-(+)-3-isopropyl-5-acetoxymethyloxazolidin-2-one having a specific optical rotatory power of $[\alpha]_D^{20}$ +32.4° (C = 1.0, chloroform).

The above (S)-(+)-3-isopropyl-5-acetoxymethyloxazolidin-2-one was further hydrolyzed to give 2.5 g of (S)-(+)-3-isopropyl-5-hydroxymethyloxazolidin-2-one having a specific optical rotatory power of $[\alpha]_D^{20}$ +36.9° (C = 1.0, chloroform).

Examples 104 to 112

The procedure of Example 101 was repeated except that the substrate and the kinds of animal tissues were changed as shown in Table 12, respectively. The results are shown in Table 12.

Table 12

| Example | Origin of enzyme | Substrate (R,S)-(±)-3-alkyl-5-acyloxy-methyloxazolidin-2-one (R,S)-(±)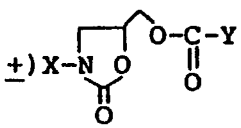 | | Product (S)-(+)-3-alkyl-5-hydroxy-methyloxazolidin-2-one (S)-(+)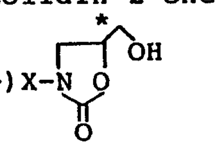 | |
|---|---|---|---|---|---|
| | | X | Y | Yield g | $[\alpha]_D^{20}$ (C=1.0, chloroform) |
| 104 | hog liver | isopropyl | ethyl | 2.8 | +39.1° |
| 105 | " | t-butyl | methyl | 2.7 | +31.6° |
| 106 | " | " | ethyl | 2.6 | +32.1° |
| 107 | rat liver | isopropyl | methyl | 2.9 | +38.8° |
| 108 | " | " | ethyl | 2.5 | +38.2° |
| 109 | " | t-butyl | methyl | 2.6 | +31.5° |
| 110 | " | " | ethyl | 2.7 | +30.8° |
| 111 | rabbit liver | isopropyl | methyl | 2.7 | +38.5° |
| 112 | " | t-butyl | methyl | 2.5 | +31.3° |

Example 113

A crude enzyme was prepared in the same manner as in Example 101 except that a hog pancreas was used instead of a hog liver, and fraction being precipitated in an ethanol concentration of 22 to 27% (v/v) was recovered. Into 100 ml of 0.1 M phosphate buffer of pH 7.0 was dissolved 5 g of the above precipitated fraction as a crude enzme and added 10 g of (R,S)-(±)-3-isopropyl-5-acetoxymethyloxazolidin-2-one as a substrate.

The resulting mixture was treated in the same manner as in Example 101 to give 2.2 g of (S)-(+)-3-isopropyl-5-hydroxymethyloxazolidin-2-one having a specific optical rotatory power of $[\alpha]_D^{20}$ +29.8°C (C = 1.0, chloroform).

## Claims

1. A process for producing an (S)-(+)-3-alkyl-5-acyloxymethyloxazolidin-2-one which comprises subjecting an (R,S)-(±)-3-alkyl-5-acyloxymethyloxazolidin-2-one having the formula [(R,S)—II]

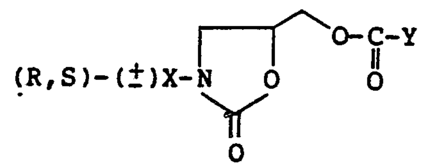

[(R,S)-II]

wherein X is a lower alkyl group having 1 to 6 carbon atoms and Y is a substituted or unsubstituted alkyl group, an alkenyl group, or a substituted or unsubstituted aromatic hydrocarbon group, to the action of a microorganism or an enzyme derived from said microorganism or an animal tissue, said microorganism and enzyme having a stereoselective esterase activity capable of asymmetrically hydrolyzing the compound [(R,S)—II] so as to produce substantially an (R)-(−)-3-alkyl-5-hydroxymethyloxazolidin-2-one having the formula [(R)—I]:

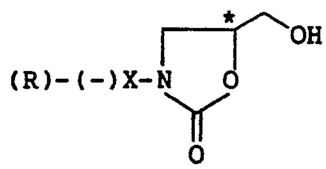

[(R)-I]

wherein X is as defined above,
and isolating the unreacted (S)-(+)-3-alkyl-5-acyloxymethyloxazolidin-2-one having the formula [(S)—II]:

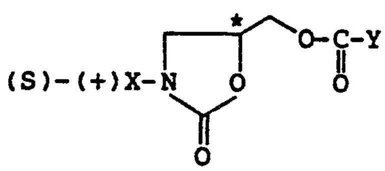

[(S)-II]

wherein X and Y are as defined above,
from the produced compound [(R—I] and organic acid.

2. The process of Claim 1, wherein X is t-butyl group or isopropyl group.

3. The process of Claim 1, wherein Y is an aryl group, a substituted aryl group, an aralkyl group or a substituted aralkyl group.

4. The process of Claim 1, wherein said microorganism belongs to a genus selected from the group consisting of the genus *Enterobacter, Klebsiella, Micrococcus, Citrobacter, Escherichia, Pseudomonas, Staphylococcus, Alcaligenes, Achromobacter, Streptomyces, Candida, Debaryomyces, Endomyces, Hansenula, Geotrichum, Kluyveromyces, Metschnikowia* and *Pichia.*

5. The process of Claim 1, wherein said microorganism belongs to a species selected from the group consisting of *Enterobacter aerogenes, Enterobacter cloacae, Klebsiella pneumoniae, Micrococcus luteus, Citrobacter freundii, Escherichia coli, Pseudomonas cruciviae, Pseudomonas fluorescens, Staphylococcus aureus, Alcaligenes faecilis, Achromobacter parvulus, Streptomyces flavovirens, Streptomyces aureus, Candida pseudotropicalis, Candida kefyr, Debaryomyces hansenii, Endomyces geotrichum, Hansenula anomala, Hansenula minuta, Geotrichum candidum, Kluyveromyces fragilis, Metschnikowia pulcherrima, Pichia farinosa* and *Pichia membranaefaciens.*

6. The process of Claim 1, wherein the microorganism is cultured and reacted in a medium containing the compound [(R,S)—II] at a pH of from 4 to 8.

7. The process of Claim 1, wherein the compound [(R,S)—II] is subjected to the action of a cultured broth obtained by culturing the microorganism in a culture medium or a cell suspension prepared by separating cells from said cultured broth.

8. The process of Claim 7, wherein the microorganism is cultured at a pH of from 3 to 11, and the compound [(R,S)—II] is subjected to the action of the cultured broth or the cell suspension at a pH of from 4 to 8.

9. The process of Claim 1, wherein said enzyme is prepared from a cell-free extract obtained by homogenizing the cells of said microorganism, or a partially purified enzyme obtained by fractionating said cell-free extract with ammonium sulfate.

10. The process of Claim 1, wherein said animal is a mammal or a bird.

11. The process of Claim 1, wherein said animal tissue is a liver or a pancreas.

12. The process of Claim 1, wherein said enzyme is a crude enzyme obtained by fractionating a homogenate of the animal tissue with a member selected from the group consisting of ethanol, aceteone, ammonium sulfate and an ion exchange resin.

13. The process of Claim 1, wherein the compound [(R,S)—II] is subjected to the action of said enzyme at a temperature of from 10° to 50°C and a pH of from 4 to 8.

14. The process of Claim 1, wherein wherein the compound [(S)—II] is isolated from the produced compound [(R)—I] and organic acid by extracting the reaction mixture with an organic solvent.

15. The process of Claim 1, wherein the compound [(S)—II] is separated from the produced compound [(R)—I] and organic acid by subjecting the reaction mixture to column chromatography.

16. The process of Claim 1, wherein the [(S)—II] is isolated from the produced compound [(R)—I] and organic acid by subjecting the reaction mixture to fractional distillation.

17. A process for preparing an (S)-(+)-3-alkyl-5-hydroxymethyloxazolidin-2-one which comprises subjecting an (R,S)-(±)-3-alkyl-5-acyloxymethyloxazolidin-2-one having the formula [(R,S)—II]:

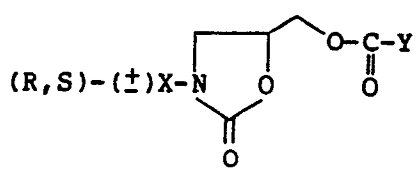

[(R,S)-II]

wherein X is a lower alkyl group having 1 to 6 carbon atoms and Y is a substituted or unsubstituted alkyl group, an alkenyl group, or a substituted or unsubstituted aromatic hydrocarbon group, to the action of a microorganism or an enzyme derived from said microorganism or an animal tissue, said microorganism and enzyme having a stereoselective esterase activity capable of asymmetrically hydrolyzing the compound [(R,S)—II] so as to produce substantially an (R)-(−)-3-alkyl-5-hydroxymethyloxazolidin-2-one having the formula [(R)—I]:

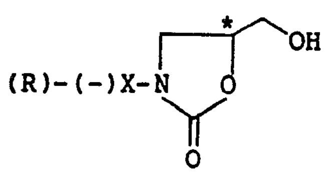

[(R)-I]

wherein X is as defined above,
isolating the unreacted (S)-(+)-3-alkyl-5-acyloxymethyloxazolidin-2-one having the formula [(S)—II]:

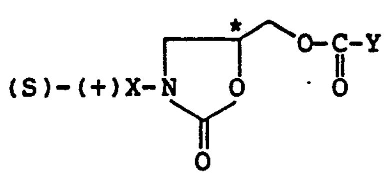

[(S)-II]

wherein X and Y are as defined above,
from the produced compound [(R)—I] and organic acid, and hydrolyzing the compound [(S)—II] to give the (S)-(+)-3-alkyl-5-hydroxymethyloxazolidin-2-one having the formula [(S)—I]:

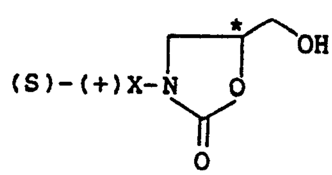

[(S)-I]

wherein X is as defined above.

18. The process of Claim 17, wherein X is t-butyl or isopropyl group.

19. The process of Claim 17, wherein Y is an aryl group, a substituted aryl group, an aralkyl group or a substituted aralkyl group.

20. The process of Claim 17, wherein said microorganism belongs to a genus selected from the group consisting of the genus *Enterobacter, Klebsiella, Micrococcus, Citrobacter, Escherichia, Pseudomonas, Staphylococcus, Alcaligenes, Achromobacter, Streptomyces, Candida, Debaryomyces, Endomyces, Hansenula, Geotrichum, Kluyveromyces, Metschnikowia* and *Pichia.*

21. The process of Claim 17, wherein said microorganism belongs to a species selected from the group consisting of *Enterobacter aerogenes, Enterobacter cloacae, Klebsiella pneumoniae, Micrococcus luteus, Citrobacter freundii, Escherichia coli, Pseudomonas cruciviae, Pseudomonas fluorescens, Staphylococcus aureus, Alcaligenes faecalis, Achromobacter parvulus, Streptomyces flavovirens, Streptomyces aureus, Candida pseudotropicalis, Candida kefyr, Debaromyces hansenii, Endomyces geotrichum, Hansenula anomala, Hansenula minuta, Geotrichum candidum, Kluyveromyces fragilis, Metschnikowia pulcherrima, Pichia farinosa* and *Pichia membranaefaciens.*

22. The process of Claim 17, wherein the microorganism is cultured and reacted in a medium containing the compound [(R,S)—II] at a pH of from 4 to 8.

23. The process of Claim 17, wherein the compound [(R,S)—II] is subjected to the action of a cultured broth obtained by culturing the microorganism in a culture medium or a cell suspension prepared by separating cells from said cultured broth.

24. The process of Claim 23, wherein said microorganism is cultured at a pH of from 3 to 11, and the compound [(R,S)—II] is subjected to the action of the cultured broth or the cell suspension at a pH of from 4 to 8.

25. The process of Claim 17, wherein said enzyme is prepared from a cell-free extract obtained by homogenizing the cells of said microorganism, or a partially purified enzyme obtained by fractionating said cell-free extract with ammonium sulfate.

26. The process of Claim 17, wherein said animal is a mammal or a bird.

27. The process of Claim 17, wherein said animal tissue is a liver or a pancreas.

28. The process of Claim 17, wherein said enzyme is a crude enzyme obtained by fractionating a homogenate of the animal tissue with a member selected from the group consisting of ethanol, acetone, ammonium sulfate and an ion exchange resin.

29. The process of Claim 17, wherein the compound [(R,S)—II] is subjected to the action of said enzyme at a temperature of from 10° to 50°C and a pH of from 4 to 8.

30. The process of Claim 17, wherein the compound [(S)—II] is isolated from the produced compound [(R)—I] and organic acid by extracting the reaction mixture with an organic solvent.

31. The process of Claim 17, wherein the compound [(S)—II] is separated from the produced compound [(R)—I] and organic acid by subjecting the reaction mixture to column chromatography.

32. The process of Claim 17, wherein the [(S)—II] is isolated from the produced compound [(R)—I] and organic acid by subjecting the reaction mixture to fractional distillation.

33. The process of Claim 17, wherein the compound [(S)—I] is prepared by chemically hyrolyzing the compound [(S)—II] at a pH of from 8 to 14.

34. The process of Claim 17, wherein the compound [(S)—I] is prepared by enzymatically hydrolyzing the compound [(S)—II] with an hydrolase at a pH of from 4 to 8.

35. (R,S)-(±)-3-alkyl-5-acyloxymethyloxazolidin-2-one having the formula [(R,S)-II]

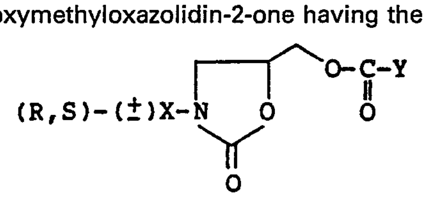

[(R,S)-II]

wherein X is a lower alkyl group having 1 to 6 carbon atoms and Y is a substituted or unsubstituted alkyl group, an alkenyl group, or a substituted or unsubstituted aromatic hydrocarbon group.

**Patentansprüche**

1. Verfahren zur Herstellung eines (S)(+)-3-Alkyl-5-acyloxymethyloxazolidin-2-ons, welches umfaßt das Unterwerfen eines (R,S)-(±)-3-Alkyl-5-acyloxymethyloxazolidin-2-ons mit der Formel [(R,S)-II]

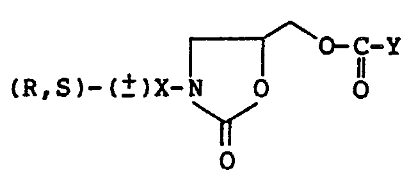

[(R,S)-II]

worin X eine niedere Alkylgruppe mit 1 bis 6 Kohlenstoffatomen ist und Y eine substituierte oder unsubstituierte Alkylgruppe, eine Alkenylgruppe oder eine substituierte oder unsubstituierte aromatische Kohlenwasserstoffgruppe ist, der Einwirkung eines Mikroorganismus oder eines von diesem

Mikroorganismus oder Tiergewebe abgeleiteten Enzyms, wobei der Mikroorganismus und das Enzym stereoselektive Esterase-Aktivität zur asymmetrischen Hydrolyse der Verbindung [(R,S)-II] aufweisen, um im wesentlichen (R)(−)-3-Alkyl-5-hydroxymethyloxazolidin-2-on mit der Formel [(R)-I] herzustellen:

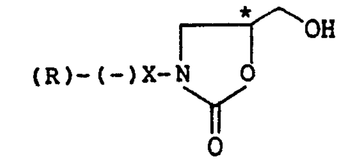

[(R)-I]

worin X wie oben definiert ist,
und die Isolierung des unreagierten (S)(+)-3-Alkyl-5-acyloxymethyloxazolidin-2-ons mit der Formel [(S)-II]

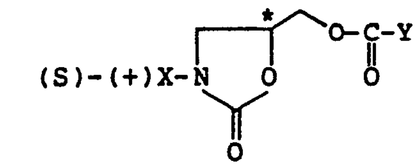

[(S)-II]

worin X und Y wie oben definiert sind,
von der hergestellten Verbindung [(R)-I] und der organischen Säure.

2. Verfahren nach Anspruch 1, worin X eine t-Butylgruppe oder Isopropylgruppe ist.

3. Verfahren nach Anspruch 1, worin Y eine Arylgruppe, eine substituierte Arylgruppe, eine Aralkylgruppe oder eine substituierte Aralkylgruppe ist.

4. Verfahren nach Anspruch 1, worin der Mikroorganismus zu einer aus der Gruppe der Familien *Enterobacter, Klebsiella, Micrococcus, Citrobacter, Escherichia, Pseudomonas, Staphylococcus, Alcaligenes, Achromobacter, Streptomyces, Candida, Debaryomyces, Endomyces, Hansenula, Geotrichum, Kluyveromyces, Metschnikowia* und *Pichia* ausgewählten Familie gehört.

5. Verfahren nach Anspruch 1, worin der Mikroorganismus zu einer aus der Gruppe der Arten *Enterobacter aerogenes, Enterobacter cloacae, Klebsiella pneumoniae, Micrococcus luteus, Citrobacter freundii, Escherichia coli, Pseudomonas cruciviae, Pseudomonas fluorescens, Staphylococcus aureus, Alcaligenes faecilis, Achromobacter parvulus, Streptomyces flavovirens, Streptomyces aureus, Candida pseudotropicalis, Candida kefyr, Debaryomyces hansenii, Endomyces geotrichum, Hansenula anomala, Hansenula minuta, Geotrichum candidum, Kluyveromyces fragilis, Metschnikowia pulcherrima, Pichia farinosa* und *Pichia membranaefaciens* ausgewählten Art gehört.

6. Verfahren nach Anspruch 1, worin der Mikroorganismus in einem die Verbindung [(R,S)-II] enthaltenden Medium bei einem pH von 4 bis 8 kultiviert und umgesetzt wird.

7. Verfahren nach Anspruch 1, worin die Verbindung [(R,S)-II] der Einwirkung einer kultivierten Brühe, erhalten durch Kultivierung des Mikroorganismus in einem Kulturmedium, oder einer Zellsuspension, hergestellt durch Abtrennen von Zellen aus dieser Kulturbrühe, unterworfen wird.

8. Verfahren nach Anspruch 7, worin der Mikroorganismus bei einem pH von 3 bis 11 kultiviert wird und die Verbindung [(R,S)-II] der Einwirkung kultivierten Brühe oder der Zellsuspension bei einem pH von 4 bis 8 unterworfen wird.

9. Verfahren nach Anspruch 1, worin das Enzym aus einem zellfreien Extrakt, erhalten durch Homogenisieren von Zellen des Mikroorganismus, oder aus einem partiell gereinigten Enzym, erhalten durch Fraktionierung des zellfreien Extrakts mit Ammoniumsulfat, hergestellt wird.

10. Verfahren nach Anspruch 1, worin das Tier ein Säugetier oder ein Vogel ist.

11. Verfahren nach Anspruch 1, worin das Tiergewebe Leber oder Pankreas ist.

12. Verfahren nach Anspruch 1, worin das Enzym ein durch Fraktionieren eines Homogenisats von Tiergewebe mit einem Glied, ausgewählt aus der Gruppe, bestehend aus Ethanol, Aceton, Ammoniumsulfat und einem Ionenaustauscherharz, erhaltenes Rohenzym ist.

13. Verfahren nach Anspruch 1, worin die Verbindung [(R,S)-II] der Einwirkung des Enzyms bei einer Temperatur von 10° bis 50°C und einem pH von 4 bis 8 unterworfen wird.

14. Verfahren nach Anspruch 1, worin die Verbindung [(S)-I] durch Extraktion der Reaktionsmischung mit einem organischen Lösungsmittel von der hergestellten Verbindung [(R)-I] und der organischen Säure isoliert wird.

15. Verfahren nach Anspruch 1, worin die Verbindung [(S)-II] durch Unterwerfen der Reaktionsmischung der Säulenchromatgraphie von der hergestellen Verbindung [(R)-I] und der organischen Säure getrennt wird.

16. Verfahren nach Anspruch 1, worin die Verbindung [(S)-II] durch Unterwerfen der Reaktionsmischung der fraktionierten Destillation von der hergestellten Verbindung [(R)-I] und der organischen Säure isoliert wird.

17. Verfahren zur Herstellung eines (S)(+)-3-Alkyl-5-hydroxymethyloxazolidin-2-ons, welches umfaßt das Unterwerfen eines (R,S)-(±)-3-Alkyl-5-acyloxymethyloxazolidin-2-ons mit der Formel [(R,S)-II]

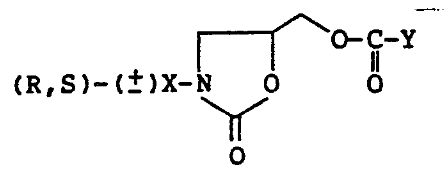

[(R,S)-II]

worin X eine niedere Alkylgruppe mit 1 bis 6 Kohlenstoffatomen ist und Y eine substituierte oder unsubstituierte Alkylgruppe, eine Alkenylgruppe oder eine substituierte oder unsubstituierte aromatische Kohlenwasserstoffgruppe ist, der Einwirkung eines Mikroorganismus oder eines von diesem Mikroorganismus oder Tiergewebe geleiteten Enzyms, wobei der Mikroorganismus und das Enzym stereoselektive Esterase-Aktivität fur die asymmetrische Hydrolyse der Verbindung [(R,S)-II] aufweisen, um im wesentlichen ein (R)(−)-3-Alkyl-5-hydroxymethyloxazolidin-2-on mit der Formel [(R)-I] herzustellen:

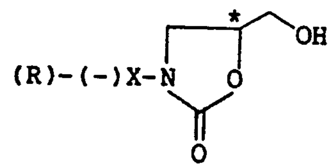

[(R)-I]

worin X wie oben definiert ist,
Isolieren des unreagierten (S)(+)-3-Alkyl-5-acyloxymethyloxazolidin-2-ons mit der Formel [(S)-II]

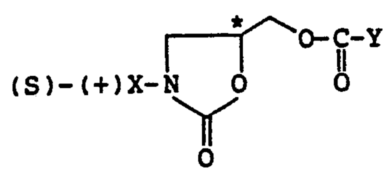

[(S)-II]

worin X und Y wie oben definiert sind,
von der hergestellten Verbindung [(R)-I] und der organischen Säure und Hydrolysieren der Verbindung [(S)-II] under Erhalt von (S)(+)-3-Alkyl-5-hydroxymethyloxazolidin-2-on mit der Formel [(S)-I]

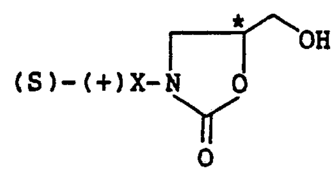

[(S)-I]

worin X wie oben definiert ist.

18. Verfahren nach Anspruch 17, worin X eine t-Butylgruppe oder Isopropylgruppe ist.

19. Verfahren nach Anspruch 17, worin Y eine Arylgruppe, eine substituierte Arylgruppe, eine Aralkylgruppe oder eine substituierte Aralkylgruppe ist.

20. Verfahren nach Anspruch 17, worin der Mikroorganismus zu einer aus der Gruppe der Familien *Enterobacter, Klebsiella, Micrococcus, Citrobacter, Escherichia, Pseudomonas, Staphylococcus, Alcaligenes, Achromobacter, Streptomyces, Candida, Debaryomyces, Endomyces, Hansenula, Geotrichum, Kluyveromyces, Metschnikowia* und *Pichia* ausgewählten Familie gehört.

21. Verfahren nach Anspruch 17, worin der Mikroorganismus zu einer aus der Gruppe der Arten *Enterobacter aerogenes, Enterobacter cloacae, Klebsiella pneumoniae, Micrococcus luteus, Citrobacter freundii, Escherichia coli, Pseudomonas cruciviae, Pseudomonas fluorescens, Staphylococcus aureus, Alcaligenes faecalis, Achromobacter parvulus, Streptomyces flavovirens, Streptomyces aureus, Candida pseudotropicalis, Candida kefyr, Debaromyces hansenii, Endomyces geotrichum, Hansenula anomala, Hansenula minuta, Geotrichum candidum, Kluyveromyces fragilis, Metschnikowia pulcherrima, Pichia farinosa* und *Pichia membranaefaciens* ausgewählten Art gehört.

22. Verfahren nach Anspruch 17, worin der Mikroorganismus in einem die Verbindung [(R,S)-II] enthaltenden Medium bei einem pH von 4 bis 8 kultiviert und umgesetzt wird.

23. Verfahren nach Anspruch 17, worin die Verbindung [(R,S)-II] der Einwirkung einer kultivierten Brühe, erhalten durch Kultivierung des Mikroorganismus in einem Kulturmedium, oder einer Zellsuspension, hergestellt durch Abtrennen von Zellen aus dieser Kulturbrühe, unterworfen wird.

24. Verfahren nach Anspruch 23, worin der Mikroorganismus bei einem pH von 3 bis 11 kultiviert wird und die Verbindung [(R,S)-II] der Einwirkung kultivierten Brühe oder der Zellsuspension bei einem pH von 4 bis 8 unterworfen wird.

25. Verfahren nach Anspruch 17, worin das Enzym aus einem zellfreien Extrakt, erhalten durch Homogenisieren von Zellen des Mikroorganismus, oder aus einem partiell gereinigten Enzym, erhalten durch Fraktionierung des zellfreien Extrakts mit Ammoniumsulfat, hergestellt wird.

26. Verfahren nach Anspruch 17, worin das Tier ein Säugetier oder ein Vogel ist.

27. Verfahren nach Anspruch nach Anspruch 17, worin das Tiergewebe Leber oder Pankreas ist.

28. Verfahren nach Anspruch 17, worin das Enzym ein durch Fraktionieren eines Homogenisats von Tiergewebe mit einem Glied, ausgewählt aus der Gruppe, bestehend aus Ethanol, Aceton, Ammoniumsulfat und einem Ionenaustauscherharz, erhaltenes Rohenzym ist.

29. Verfahren nach Anspruch 17, worin die Verbindung [(R,S)-II] der Einwirkung des Enzyms bei einer Temperatur von 10° bis 50°C und einem pH von 4 bis 8 unterworfen wird.

30. Verfahren nach Anspruch 17, worin die Verbindung [(S)-II] durch Extraktion der Reaktionsmischung mit einem organischen Lösungsmittel von der hergestellten Verbindung [(R)-I] und der organischen Säure isoliert wird.

31. Verfahren nach Anspruch 17, worin die Verbindung [(S)-II] durch Unterwerfen der Reaktionsmischung der Säulenchromatgraphie von der hergestellen Verbindung [(R)-I] und der orgnischen Säure getrennt wird.

32. Verfahren nach Anspruch 17, worin die Verbindung [(S)-II] durch Unterwerfen der Reaktionsmischung der fraktionierten Destillation von der hergestellten Verbindung [(R)-I] und der organischen Säure isolieret wird.

33. Verfahren nach Anspruch 17, worin die Verbindung [(S)-I] durch chemische Hydrolyse der Verbindung [(S)-II] bei einem pH von 8 bis 14 hergestellt wird.

34. Verfahren nach Anspruch 17, worin die Verbindung [(S)-I] durch enzymatische Hydrolyse der Verbindung [(S)-II] mit einer Hydrolase bei einem pH von 4 bis 8 hergestellt wird.

35. (R,S)-(±)-3-Alkyl-5-acyloxymethyloxazolidin-2-ons mit der Formel [(R,S)-II]

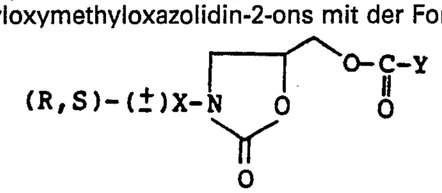

[(R,S)-II]

worin X eine niedere Alkylgruppe mit 1 bis 6 Kohlenstoffatomen ist und Y eine substituierte oder unsubstituierte Alkylgruppe, eine Alkenylgruppe oder eine substituierte oder unsubstituierte aromatische Kohlenwasserstoffgruppe.

## Revendications

1. Procédé de production d'une (S)-(+)-3-alkyl-5-acyloxyméthyloxazolidin-2-one qui comprend les étapes qui consistent à soumettre une (R,S)-(±)-3-alkyl-5-acyloxyméthyloxazolidine-2-one de formule [(R,S)-II]

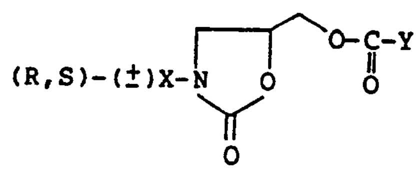

[(R,S)-II]

dans laquelle X est un groupe alkyle inférieur ayant 1 à 6 atomes de carbone et Y est un groupe alkyle substitué ou non substitué, un groupe alcényle ou un groupe hydrocarboné aromatique substitué ou non substitué, à l'action d'un microorganisme ou d'une enzyme dérivée dudit microorganisme ou d'un tissu animal, ledit microorganisme et ladite enzyme ayant une activité d'estérase stéréosélective capable d'hydrolyser de façon asymétrique le composé [(R,S)-II], de manière à produire essentiellement une (R)-(−)-3-alkyl-5-hydroxymethyloxazolidin-2-one ayant pour formule [(R)-I]:

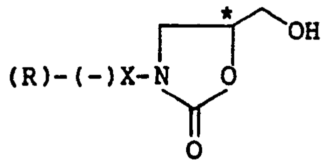

[(R)-I]

dans laquelle X est tel défini ci-dessus, et à isoler la (S)-(+)-3-alkyl-5-acyloxyméthyloxazolidine-2-one n'ayant pas réagi, de formule [(S)-II]:

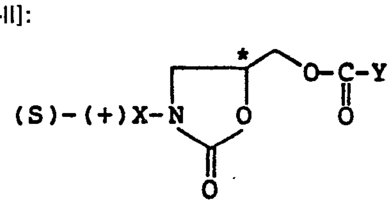

[(S)-II]

dans laquelle X et Y sont tels que définis ci-dessus, du composé [(R)-I] produit et de l'acide organique.

2. Procédé selon la revendication 1, dans lequel X est un groupe t-butyle ou un groupe isopropyle.

3. Procédé selon la revendication 1, dans lequel Y est un groupe aryle, un groupe aryle substitué, un groupe arylalkyle ou un groupe arylalkyle substitué.

4. Procédé selon la revendication 1, dans lequel le microorganisme appartient à une gène choisi dans le groupe constitué par les genres *Enterobacter, Klebsiella, Micrococcus, Citrobacter, Escherichia, Pseudomonas, Staphylococcus, Alcaligenes, Achromobacter, Streptomyces, Candida, Debaryomyces, Endomyces, Hansenula, Geotrichum, Kluyveromyces, Metschnikowia* et *Pichia.*

5. Procédé selon la revendication 1, dans lequel le microorganisme appartient à une espèce choisie dans le groupe constitué par *Enterobacter aerogenes, Enterobacter cloacae, Klebsiella pneumoniae, Micrococcus luteus, Citrobacter freundii, Escherichia coli, Pseudomonas cruciviae, Pseudomonas fluorescens, Staphylococcus aureus, Alcaligenes faecilis, Achromobacter parvulus, Streptomyces flavovirens, Streptomyces aureus, Candida pseudotropicalis, Candida kefyr, Debaryomyces hansenii, Endomyces geotrichum, Hansenula anomala, Hansenula minuta, Geotrichum candidum, Kluyveromyces fragilis, Metschnikowia pulcherrima, Pichia farinosa* et *Pichia membranaefaciens.*

6. Procédé selon la revendication 1, dans lequel le microorganisme est cultivé et est mis en réaction dans un milieu contenant le composé [(R,S)-II] à un pH de 4 à 8.

7. Procédé selon la revendication 1, dans lequel le composé [(R,S)-II] est soumis à l'action d'un bouillon de culture obtenu par culture du microorganisme dans le milieu de culture ou dans une suspension cellulaire préparée par séparation des cellules dudit bouillon de culture.

8. Procédé selon la revendication 7, dans lequel le microorganisme est cultivé à un pH de 3 à 11, et le composé [(R,S)-II] est soumis à l'action du bouillon de culture ou de la suspension cellulaire à un pH de 4 à 8.

9. Procédé selon la revendication 1, dans lequel ladite enzyme est préparée à partir d'un extrait acellulaire obtenu par homogénéisation des cellules dudit microorganisme, ou d'une enzyme partiellement purifiée obtenue par fractionnement de l'extrait acellulaire avec du sulfate d'ammonium.

10. Procédé selon la revendication 1, dans lequel ledit animal est une mammifère ou un oiseau.

11. Procédé selon la revendication 1, dans lequel ladite tissu animal est un foie ou un pancréas.

12. Procédé selon la revendication 1, dans lequel ladite enzyme est enzyme brute obtenue par fractionnement d'un homogénat du tissue animal avec un élément choisi dans le group constitué l'éthanol, l'acétone, le sulfate d'ammonium et un résine échangeuse d'ions.

13. Procédé selon la revendication 1, dans lequel le composé [(R,S)-II] est soumis à l'action de ladite enzyme à une température de 10° à 50°C et à une pH de 4 à 8.

14. Procédé selon la revendication 1, dans lequel le composé [(S)-II] est isolé du composé produit [(R)-I] et de l'acide organique par extraction du mélange réactionnel avec un solvant organique.

15. Procédé selon la revendication 1, dans lequel le composé [(S)-II] est séparé du composé produit [(R)-I] et de l'acide organique par chromatographie sur colonne du mélange réactionnel.

16. Procédé selon la revendication 1, dans lequel le composé [(S)-II] est isolè du composé produit [(R)-I] et de l'acide organique par distillation fractionné du mélange réactionnel.

17. Procédé de préparation d'une (S)-(+)-3-alkyl-5-hydroxyméthyloxazolidin-2-one qui comprend les étapes qui consistent à soumettre une (R,S)-(±)-3-alkyl-5-acyloxyméthyloxazolidin-2-one de formule [(R,S)-II]

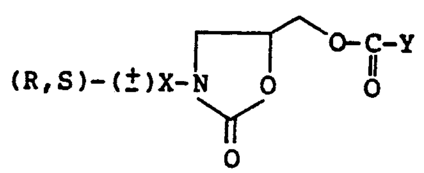

[(R,S)-II]

dans laquelle X est un groupe alkyle inférieur ayant 1 à 6 atomes de carbone et Y est un groupe alkyle substitué ou non substitué, un groupe alcényle ou un groupe hydrocarboné aromatique substitué ou non substitué, à l'action d'un microorganisme ou d'une enzyme dérivée dudit microorganisme ou d'un tissu animal, ledit microorganisme et ladite enzyme ayant une activité a l'estérase stéréosélective capable d'hydrolyser de façon asymétrique le composé [(R,S)-II], de manière à produire essentiellement une (R)-(−)-3-alkyl-5-hydroxymethyloxazolidine-2-one ayant pour formule [(R)-I]:

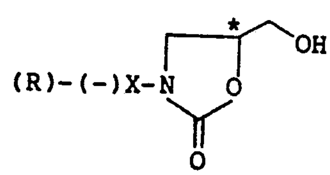

[(R)-I]

dans laquelle X est tel que défini ci-dessus, à isoler la (S)-(+)-3-alkyl-5-acyloxyméthyloxazolidin-2-one n'ayant pas réagi, de formule [(S)-II]:

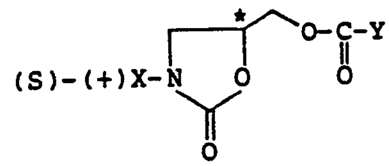

[(S)-II]

dans laquelle X et Y sont tels que définis ci-dessus, du composé [(R)-I] produit et de l'acide organique, et à hydrolyser le composé [(S)-II] pour donner la (S)-(+)-3-alkyl-5-hydroxyméthyloxazolidine-2-one de formule [(S)-I]:

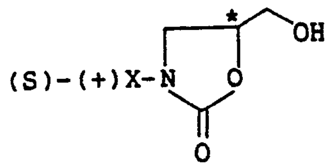

[(S)-I]

dans laquelle X est tel que défini ci-dessus.

18. Procédé selon la revendication 17, dans lequel X est un groupe t-butyle ou un groupe isopropyle.

19. Procédé selon la revendication 17, dans lequel Y est un groupe aryle, un groupe aryle substitué, un groupe arylalkyle ou un groupe arylalkyle substitué.

20. Procédé selon la revendication 17, dans lequel le microorganisme appartient à une gène choisi dans le groupe constitué par les genres *Enterobacter, Klebsiella, Micrococcus, Citrobacter, Escherichia, Pseudomonas, Staphylococcus, Alcaligenes, Achromobacter, Streptomyces, Candida, Debaryomyces, Endomyces, Hansenula, Geotrichum, Kluyveromyces, Metschnikowia* et *Pichia.*

21. Procédé selon la revendication 17, dans lequel le microorganisme appartient à une espèce choisie dans le groupe constitué par *Enterobacter aerogenes, Enterobacter cloacae, Klebsiella pneumoniae, Micrococcus luteus, Citrobacter freundii, Escherichia coli, Pseudomonas cruciviae, Pseudomonas fluorescens, Staphylococcus aureus, Alcaligenes faecilis, Achromobacter parvulus, Streptomyces flavovirens, Streptomyces aureus, Candida pseudotropicalis, Candida kefyr, Debaryomyces hansenii, Endomyces geotrichum, Hansenula anomala, Hansenula minuta, Geotrichum candidum, Kluyveromyces fragilis, Metschnikowia pulcherrima, Pichia farinosa* et *Pichia membranaefaciens.*

22. Procédé selon la revendication 17, dans lequel le microorganisme est cultivé et est mis en réaction dans un milieu contenant la composé [(R,S)-II] à un pH de 4 à 8.

23. Procédé selon la revendication 17, dans lequel le composé [(R,S)-II] est soumis à l'action d'un bouillon de culture obtenu par culture de microorganisme dans le milieu de culture ou dans une suspension cellulaire préparée par séparation des cellules dudit bouillon de culture.

24. Procéde selon la revendication 23, dans lequel le microorganisme est cultivé à un pH de 3 à 11, et le composé [(R,S)-II] est soumis à l'action d'un bouillon de culture ou de la suspension cellulaire à un pH de 4 à 8.

25. Procédé selon la revendication 17, dans lequel ladite enzyme est préparé à partir d'un extrait acellulaire obtenu par homogénéisation des cellules dudit microorganisme, ou d'une enzyme partiellement purifiée obtenue par fractionement de l'extrait acellulaire avec du sulfate d'ammonium.

26. Procédé selon la revendication 17, dans lequel ledit animal est une mammifère ou un oiseau.

27. Procédé selon la revendication 17, dans lequel ledit tissu animal est un foie ou un pancréas.

28. Procédé selon la revendication 17, dans lequel ladite enzyme est enzyme brute obtenue par fractionnement d'un homogénat du tissue animal avec un élément choisi dans le group constitué l'éthanol, l'acétone, le sulfate d'ammonium et un résine échangeuse d'ions.

29. Procédé selon la revendication 17, dans lequel le composé [(R,S)-II] est soumis à l'action de ladite enzyme à une température de 10° à 50°C et à une pH de 4 à 8.

30. Procédé selon la revendication 17, dans lequel le composé [(S)-II] est isolé du composé produit [(R)-I] et de l'acide organique par extraction du mélange réactionnel avec un solvant organique.

31. Procédé selon la revendication 17, dans lequel le composé [(S)-II] est séparé du composé produit [(R)-I] et de l'acide organique par chromatographie sur colonne du mélange réactionnel.

32. Procédé selon la revendication 17, dans lequel le composé [(S)-II] est isolè du composé produit [(R)-I] et de l'acide organique par distillation fractionnée du mélange réactionnel.

33. Procédé selon la revendication 17, dans lequel le compoé [(S)-I] est préparé par hydrolyse chimique du composé [(S)-II] à un pH de 8 à 14.

34. Procédé selon la revendication 17, dans lequel le composé [(S)-I] est préparé par hydrolyse enzymatique du composé [(S)-II] avec une hydrolase à un pH 4 à 8.

35. (R,S)-(±)-3-alkyl-5-acyloxyméthyloxazolidin-2-one de formule [(R,S)-II]

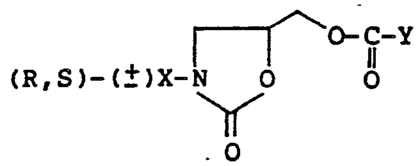

[(R,S)-II]

dans laquelle X est un groupe alkyle inférieur ayant 1 à 6 atomes de carbone et Y est un groupe alkyle substitué ou non substitué, un groupe alcényle ou un groupe hydrocarboné aromatique substitué or non substitué.